# EUROPÄISCHE PATENTSCHRIFT

(11) **EP 1 751 132 B1**
(45) Veröffentlichungstag und Bekanntmachung des Hinweises auf die Patenterteilung: **26.03.2008**
(21) Anmeldenummer: 05741763.6
(22) Anmeldetag: 17.05.2005
(51) Int. Cl.: C07D 401/04, C07D 403/04, A01N 43/54

(54) **2-SUBSTITUIERTE PYRIMIDINE UND IHRE VERWENDUNG ALS PESTIZIDE**
2-SUBSTITUTED PYRIMIDINES AND THEIR USE AS PESTICIDES
PYRIMIDINES 2-SUBSTITUEES ET LEUR UTILISATION EN TANT QUE PESTICIDES

(30) Priorität: 19.05.2004 DE 102004025363
(43) Veröffentlichungstag der Anmeldung: 14.02.2007
(73) Patentinhaber: BASF SE, 67056 Ludwigshafen (DE)
(72) Erfinder: SCHWÖGLER, Anja, 68165 Mannheim (DE); SCHIEWECK, Frank, 67258 Hessheim (DE); RHEINHEIMER, Joachim, 67063 Ludwigshafen (DE); GEWEHR, Markus, 56288 Kastellaun (DE); MÜLLER, Bernd, 67227 Frankenthal (DE); GROTE, Thomas, 67157 Wachenheim (DE); GRAMMENOS, Wassilios, 67071 Ludwigshafen (DE); HÜNGER, Udo, 55124 Mainz (DE); BLETTNER, Carsten, 68165 Mannheim (DE); SCHÄFER, Peter, 67308 Ottersheim (DE); WAGNER, Oliver, 67433 Neustadt (DE); STIERL, Reinhard, 67251 Freinsheim (DE); SCHÖFL, Ulrich, 68782 Brühl (DE); STRATHMANN, Siegfried, 67117 Limburgerhof (DE); SCHERER, Maria, 76829 Godramstein (DE)
(86) Internationale Anmeldenummer: PCT/EP2005/005333
(87) Internationale Veröffentlichungsnummer: WO 2005/113538

(56) Entgegenhaltungen:
- WO-A-02/074753
- WO-A-20/04103978

## Beschreibung

Die Erfindung betrifft 2-substituierte Pyrimidine der Formel I 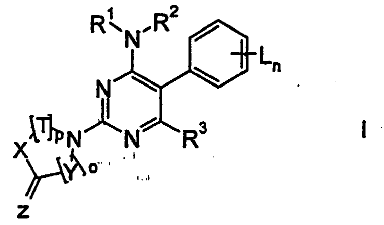 in der die Indices und die Substituenten die folgende Bedeutung haben:
- n: eine ganze Zahl von 1 bis 5;
- L: Halogen, Cyano, Cyanato (OCN), C₁-C₈-Alkyl, C₂-C₈-Alkenyl, C₂-C₈-Alkinyl, C₁-C₆-Alkoxy, C₂-C₈-Alkenyloxy, C₂-C₈-Alkinyloxy, C₃-C₆-Cycloalkyl, C₄-C₆-Cycloalkenyl, C₃-C₆-Cycloalkyloxy, C₄-C₆-Cycloalkenyloxy, Nitro, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A,
- m: 0, 1 oder 2;
- A, A', A": unabhängig voneinander Wasserstoff, C₁-C₆-Alkyl, C₂-C₆-Alkenyl, C₂-C₆-Alkinyl, C₃-C₈-Cycloalkyl, C₃-C₈-Cycloalkenyl, Phenyl, wobei die organischen Reste partiell oder vollständig halogeniert sein können oder durch Nitro, Cyana-to, Cyano oder C₁-C₄-Alkoxy substituiert sein können; oder A und A' zusammen mit den Atomen an die sie gebunden sind für einen fünf- bis sechsgliedrigen gesättigten, partiell ungesättigten oder aromatischen Heterocyclus, enthaltend ein bis vier Heteroatome aus der Gruppe O, N oder S, stehen;
wobei die aliphatischen Gruppen der Restedefinitionen von L ihrerseits partiell oder vollständig halogeniert sein oder eine bis vier Gruppen R^{u} tragen können:
R^{u} Cyano, C₁-C₆-Alkoxy, C₃-C₆-Cycloalkyl, C₂-C₈-Alkenyloxy, C₂-C₈-Alkinyloxy, C₄-C₆-Cycloalkenyl, C₃-C₆-Cycloalkyloxy, C₄-C₆-Cycloalkenyloxy
-C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A;
R¹, R²unabhängig voneinander C₁-C₆-Alkyl, C₂-C₆-Alkenyl, C₂-C₆-Alkinyl, C₃-C₆-Cycloalkyl, C₃-C₆-Halogencycloalkyl, wobei die aliphatischen Gruppen der Restedefinitionen von R¹ und R² ihrerseits partiell oder vollständig halogeniert sein oder eine bis vier Gruppen R^{v} tragen können:
R^{v} Cyano, C₃-C₆-Cycloalkyl, C₄-C₆-Cycloalkenyl, Hydroxy, C₁-C₆-Alkoxy, C₂-C₈-Alkenyloxy, C₂-C₈-Alkinyloxy, C₃-C₆-Cycloalkyloxy, C₄-C₆-Cycloalkenyloxy, C₁-C₆-Alkylthio, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A oder Phenyl, wobei der Phenylteil ein bis drei Reste ausgewählt aus der Gruppe: Halogen, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy, Cyano, Nitro, -C(=O)-A, -C(=O)-O-A, -C(=O)-N(A')A, C(A')(=N-OA), N(A')A tragen kann;
R² kann zusätzlich Wasserstoff bedeuten;
R¹ und R² können auch zusammen mit dem Stickstoffatom, an das sie gebunden sind, einen gesättigten oder ungesättigten fünf- oder sechsgliedrigen Ring bilden, der durch eine Ether -(-O-), Carbonyl -(C=O)-, Thio -(-S-), Sulfoxyl -(-S[=O]-) oder Sulfenyl -(-SO₂-) oder eine weitere Amino -(-N(R^{a})- Gruppe, wobei R^{a} Wasserstoff oder C₁-C₆-Alkyl bedeutet, unterbrochen sein und/oder einen oder mehrere Substituenten aus der Gruppe Halogen, C₁-C₆-Alkyl, C₁-C₆-Halogenalkyl und Oxy-C₁-C₃-alkylenoxy enthalten kann;
R³ Halogen, Cyano, C₁-C₄-Alkyl, C₂-C₄-Alkenyl, C₂-C₄-Alkinyl, C₃-C₆-Cycloalkyl, C₁-C₄-Alkoxy, C₃-C₄-Alkenyloxy, C₃-C₄-Alkinyloxy, C₁-C₆-Alkylthio, Di-(C₁-C₆-alkyl)amino oder C₁-C₆-Alkylamino, wobei die Alkyl, Alkenyl und Alkinylreste von R³ durch Halogen, Cyano, Nitro, C₁-C₂-Alkoxy oder C₁-C₄-Alkoxycarbonyl substituiert sein können;
X eine Gruppe -CH-R^{a}-, -N-R^{b}-, -O- oder-S-;
R^{a} Wasserstoff, Halogen; C₁-C₆-Alkyl, C₁-C₆-Alkoxy, Cyano oder C₁-C₆-Alkoxycarbonyl sein kann;
R^{b} Wasserstoff, C₁-C₆-Alkyl oder C₃-C₆-Cycloalkyl;
T eine Gruppe -CH-Ra- ;
p eine Zahl von 1 bis 4;
Y eine Gruppe -CH-R^{a}- oder -N-R^{b}-,
o 0 oder 1;
Z O, S oder eine Gruppe N(R^{c})
R^{c} Wasserstoff, C₁-C₆-Alkyl oder C₁-C₆-Alkoxy.

Außerdem betrifft die Erfindung ein Verfahren zur Herstellung dieser Verbindungen, 2-Pyrimidine enthaltende Mittel sowie deren Verwendung zur Bekämpfung pflanzenpathogener Schadpilze.

Aus WO-A 02/074753 sind fungizide Pyrimidine, die in 2-Stellung einen Heteroaryrest tragen, bekannt. Weiterhin sind aus EP-A 715 851 pharmakologisch aktive Pyrimidine bekannt, die in 2-Stellung einen 4-Methylpiperazinrest tragen, bekannt. Eine fungizide Wirkung wird in EP-A 715 851 nicht offenbart.

Die Wirkung der in 2-Stellung heteroarylsubstituierten Pyrimidine ist in vielen Fällen nicht zufriedenstellend. Daher lag als Aufgabe zugrunde, Verbindungen mit verbesserter Wirksamkeit zu finden.

Demgemäß wurden die eingangs definierten Pyrimidine der Formel I gefunden. Außerdem wurden Verfahren zu ihrer Herstellung sowie sie enthaltende Mittel zur Bekämpfung von Schadpilzen gefunden.

Die Verbindungen I können auf verschiedenen Wegen erhalten werden.
1. Beispielsweise kann von den Sulfonen der Formel II ausgegangen werden, deren Herstellung in WO-A 02/074753 oder DE 10156279.9 detailliert beschrieben ist. Durch Umsetzung der Sulfone II mit Nukleophilen der Formel III, wobei der Index p vorzugsweise 2 bis 5 bedeutet, werden die erfindungsgemäßen Lactame IA gewonnen. Die Reaktion wird in der Regel basenkatalysiert durchgeführt. Will man im ersten Schritt eine Deprotonierung der Nukleophile III erzielen, werden üblicherweise starke Baseneingesetzt. Als Basen kommen beispielsweise metallorganische Verbindungen wie: Lithiumdiisopropylamid, Buthyllithium oder Natriumhydrid in Frage. 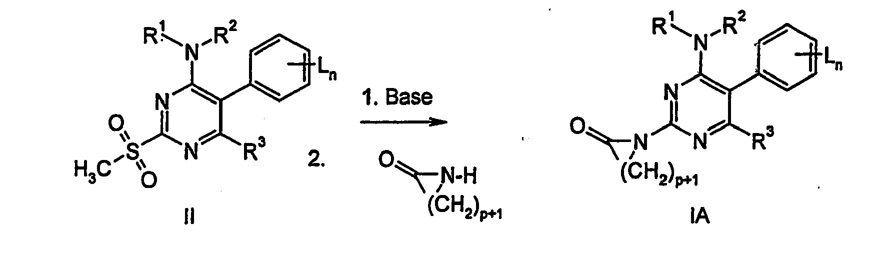
**2.** Lactamderivate der Formeln IA' und IA" (Z = S oder NR^{c}) lassen sich wie im folgenden Schema dargestellt aus den Lactamen der Formel IA (Z = O) aufbauen. 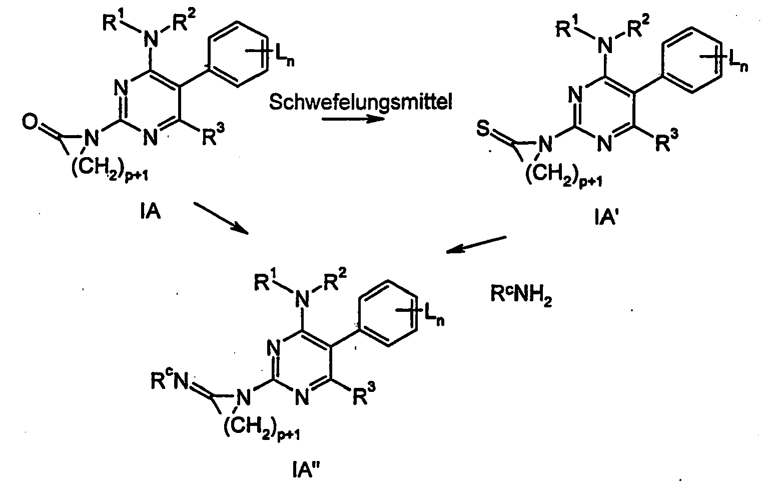 Geeignete Schwefelungsmittel sind beispielsweise Lawesson-Reagenz oder Phosphorpentasulfid. Die Schwefelungsreaktion wird vorzugsweise in einem inerten Verdünnungsmittel wie beispielsweise einem aliphatischen oder aromatischen Kohlenwasserstoff, Dimethylformamid oder N-Methylpyrrolidon durchgeführt. Die Aminierung des Thiolactams IA' zur Verbindung IA" erfolgt vorzugsweise durch Zugabe eines Überschusses an Amin NH₂R^{c}. Bei niedrigsiedenden Aminen kann die Umsetzung vorzugsweise unter Druck beispielsweise in einem Autoklaven ausgeführt werden. Unter Umständen ist es ratsam auch bei der Aminierung ein Verdünnungsmittel zuzusetzen. Als Verdünnungsmittel kommen die bei der Schwefelung bereits beschriebenen Verbindungen in Frage. Die Verbindung IA kann auch direkt zur Verbindung IA" nach literaturüblichen Methoden (säure/basekatalysiert) umgesetzt werden.
3. Verbindungen der Formel IB lassen sich beispielsweise durch Umsetzung des Hydrazins IV mit einem Chloralkylcarbonsäurechlorid, wobei der Index p 2 oder 3 bedeutet, zur Verbindung V und anschließendem Ringschluß zum Pyrazolidinon IB gewinnen. 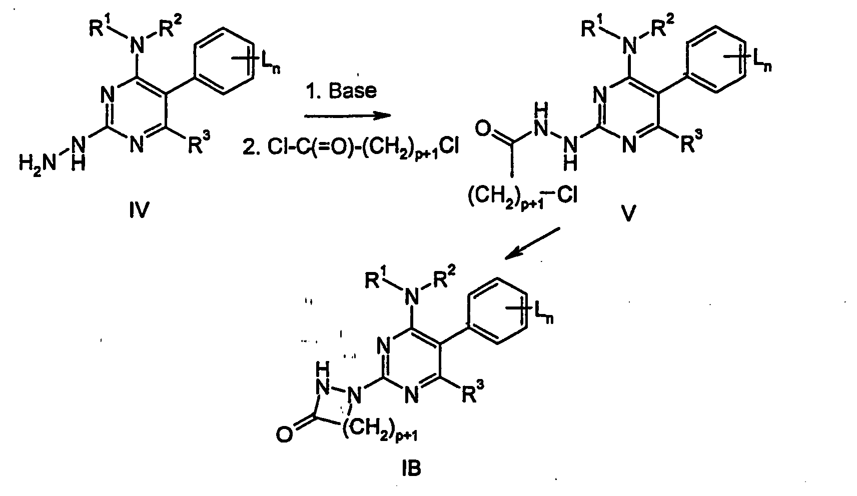
   Der Herstellung der Hydrazinverbindung IV kann beispielsweise aus dem Sulfon II durch Umsetzung mit Hydrazinhydrat gewonnen werden (s. bspw. WO-A 03/043993). Die weitere Umsetzung mit einem Chloralkylcarbonsäurechlorid erfolgt in der Regel in einem inerten Verdünnungsmittel wie einem gegebenenfalls halogenierten aliphatischen oder aromatischem Kohlenwasserstoff oder einem Ether. Als Basen kommen vorzugsweise Pyridinderivate oder tertiäre Amine in Frage. Der Ringschluß zu den Pyrazolidinonen IB kann beispielsweise in Gegenwart von metallorganischen Basen wie Butyllithium oder LDA erfolgen. Für diese Reaktion bieten sich die üblichen Lösungsmittel wie aliphatische oder cyolische Ether an.
4. Analog den unter Punkt 2 beschriebenen Methoden lassen sich ausgehend von den Pyrazolidinonen IB die entsprechenden Thioderivate IB' und Iminoderivate IB" gewinnen. 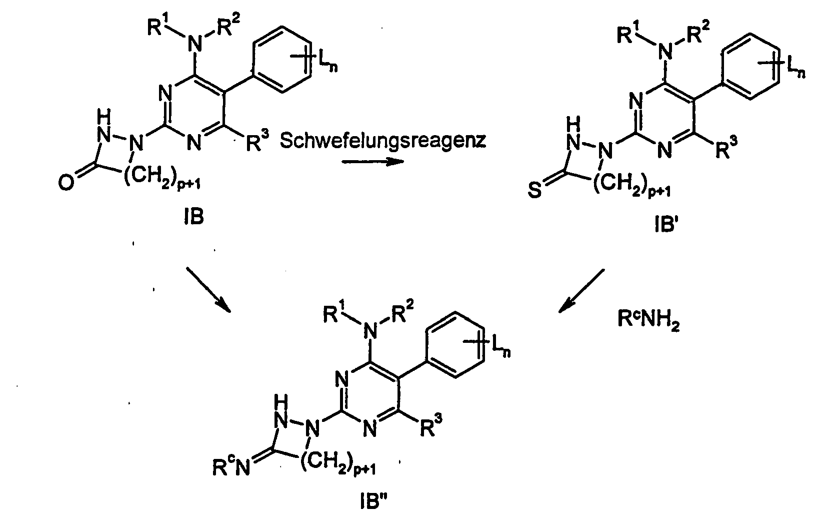
5) Sulfone der Formel II, bei denen R³ eine von Halogen verschiedene Bedeutung hat, können wie folgt gewonnen werden: 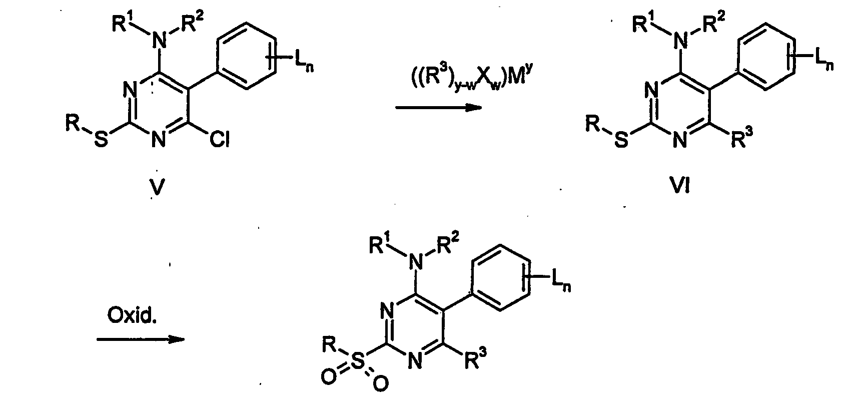 In Formel (R³)_{y-w}X_{w}-M^{y} steht M für ein Metallion der Wertigkeit Y, wie beispielsweise B, Zn, Mg, Cu oder Sn, X steht für Chlor, Brom, Iod oder Hydroxy, R³ bedeutet bevorzugt C₁-C₄-Alkyl und w steht für eine Zahl von 0 bis 3. Diese Reaktion kann beispielsweise analog folgender Methoden durchgeführt werden: J. Chem. Soc. Perkin Trans. 1, 1187 (1994), ebenda 1, 2345 (1996); WO-A 99/41255; Aust. J. Chem., Bd. 43, 733 (1990); J. Org. Chem., Bd. 43, 358 (1978); J. Chem. Soc. Chem. Commun. 866 (1979); Tetrahedron Lett., Bd. 34, 8267 (1993); ebenda, Bd. 33,413 (1992).

Die obengenannten Angaben beziehen sich insbesondere auf die Herstellung von Verbindungen, in denen R³ eine Alkylgruppe darstellt. Sofern R³ eine Cyangruppe oder einen Alkoxysubstienten bedeutet, kann der Rest R³ durch Umsetzung mit Alkalimetallcyaniden bzw. Alkalimetallalkoholaten eingeführt werden.

Bei den in den vorstehenden Formeln angegebenen Definitionen der Symbole wurden Sammelbegriffe verwendet, die allgemein repräsentativ für die folgenden Substituenten stehen:

**Halogen:** Fluor, Chlor, Brom und Jod;
**Alkyl sowie die Alkylteile von beispielsweise Alkoxy, Alkylamino, Alkoxycarbonyl:** gesättigte, geradkettige oder verzweigte Kohlenwasserstoffreste mit 1 bis 4, 6 oder 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl**: geradkettige oder verzweigte Alkylgruppen mit 1 bis 4, 6 oder 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl;
**Alkenyl**: ungesättigte, geradkettige oder verzweigte Kohlenwasserstoffreste mit 2 bis 4, 6 oder 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1propenyl und 1-Ethyl-2-methyl-2-propenyl;
**Alkadienyl**: ungesättigte, geradkettige oder verzweigte Kohlenwasserstoffreste mit 4, bis 8 Kohlenstoffatomen und zwei Doppelbindungen in beliebiger Position;
Halogenalkenyl: ungesättigte, geradkettige oder verzweigte Kohlenwasserstoffreste mit 2 bis 8 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyl**: geradkettige oder verzweigte Kohlenwasserstoffgruppen mit 2 bis 8 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Cycloalky**l: mono- oder bicyclische, gesättigte Kohlenwasserstoffgruppen mit 3 bis 6 Kohlenstoffringgliedern, z.B. C₃-C₆-Cycloalkyl wie Cyclopropyl, Cyclobutyl, Cyclopentyl, Cyclohexyl;

- **fünf- bis sechsgliedriger gesättigter, partiell ungesättigter oder aromatischer Heterocyclus, enthaltend ein bis vier Heteroatome aus der Gruppe O, N oder S**: z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-lsoxazolidinyl, 3-Isothiazolidinyl, 4-lsothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-lmidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydröthien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-lsoxazolin-3-yl, 3-Isoxazolin-3-yl, 4-lsoxazolin-3-yl, 2-lsoxazolin-4-yl, 3-lsoxazolin-4-yl, 4-lsoxazolin-4-yl, 2-Isoxazolin-5-yl, 3-Isoxazolin-5-yl, 4-Isoxazolin-5-yl, 2-lsothiazolin-3-yl, 3-lsothiazolin-3-yl, 4-lsothiazolin-3-yl, 2-lsothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydrotriazin-2-yl und 1,2,4-Hexahydrotriazin-3-yl;
- **Ringsystem, das gegebenenfalls von R¹ und R² bzw. von A und A' zusammen mit dem Stickstoff, an den sie gebunden sind, aufgespannt wird: z.B.** Pyrrolidin, Morpholin, Piperidin oder Tetrahydropyrazol.

In dem Umfang der vorliegenden Erfindung sind die (R)- und (S)-Isomere und die Racemate von Verbindungen der Formel I eingeschlossen, die chirale Zentren aufweisen.

Im folgenden werden die Ausführungsformen der Erfindung genauer beschrieben.

Im Hinblick auf die bestimmungsgemäße Verwendung der Pyrimidine der Formel I sind die folgenden Bedeutungen der Substituenten, und zwar jeweils für sich allein oder in Kombination, besonders bevorzugt: Verbindungen I werden bevorzugt, in denen R¹ für C₁-C₆-Alkyl, C₁-C₆-Halogenalkyl, C₂-C₆-Alkenyl, C₂-C₆-Alkinyl oder C₃-C₆-Cycloalkyl und R² für Wasserstoff stehen.

Insbesondere werden Verbindungen I bevorzugt, in denen R¹ für in α-Stellung verzweigtes C₁-C₆-Alkyl, C₂-C₆-Alkenyl oder C₁-C₆-Halogenalkyl steht. Besonders bevorzugt sind diejenigen Verbindungen I, in denen R¹ die zuvor genannte Bedeutung und R² Wasserstoff bedeuten.

Daneben werden Verbindungen I bevorzugt, in denen R¹ für C₁-C₄-Halogenalkyl und R² für Wasserstoff stehen.

Außerdem werden Verbindungen I bevorzugt, in denen R¹ und R² zusammen mit dem Stickstoff, an das sie gebunden sind, einen fünf- oder sechsgliedrigen Ring bilden, der durch ein Sauerstoffatom unterbrochen sein kann und einen oder zwei C₁-C₆-Alkylsubstituenten tragen kann.

Insbesondere bevorzugt sind Gruppen NR¹R² wie - insbesondere in α-Stellung - methylierte Pyrrolidine oder Piperidine. Weiterhin ist 4-Methylpiperidin bevorzugt.

Insbesondere werden Pyrimidine I bevorzugt, wobei die Substituenten L¹ bis L⁵ die folgende Bedeutung haben:
- L: Halogen, Cyano, Methyl, Methoxy, -C(=O)-O-A, -C(=O)-N(A')A, C(A')(=N-OA), N(A')A, N(A')-C(=O)-A,
A,A' unabhängig voneinander Wasserstoff, C₁-C₆-Alkyl, C₂-C₆-Alkenyl, C₂-C₆-Alkinyl, Phenyl, wobei die organischen Reste partiell oder vollständig halogeniert sein können oder durch C₁-C₄-Alkoxy substituiert sein können, oder A und A' zusammen mit den Atomen an die sie gebunden sind für einen fünf- bis sechsgliedrigen gesättigten Heterocyclus, enthaltend ein oder zwei Heteroatome aus der Gruppe O, N oder S stehen;

Außerdem werden Pyrimidine I bevorzugt, wobei die durch Lₙ substituierte Phenylgruppe für die Gruppe B 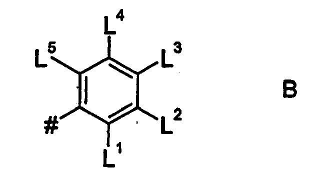 steht, worin # die Verknüpfungsstelle mit dem Pyrimidin-Gerüst ist und
- L¹: Fluor, Chlor, CH₃ oder CF₃;
- L²,L⁴: unabhängig voneinander Wasserstoff, CH₃ oder Fluor;
- L³: Wasserstoff, Fluor, Chlor, Brom, Cyano, CH₃, SCH₃, OCH₃, SO₂CH₃, CO-NH₂, CO-NHCH₃, CO-NHC₂H₅, CO-N(CH₃)₂, NH-C(=O)CH₃, N(CH₃)-C(=O)CH₃ oder COOCH₃ und
- L⁵: Wasserstoff, Fluor, Chlor oder CH₃ bedeuten.

Besonders bevorzugt werden auch Verbindungen I, in denen R³ C₁-C₄-Alkyl bedeutet, das durch Halogen substituiert sein kann.

Außerdem werden Verbindungen I besonders bevorzugt, in denen R³ für Halogen, Cyano, C₁-C₄-Alkyl oder C₁-C₄-Alkoxy steht.

Insbesondere werden Verbindungen I bevorzugt, in denen R³ Methyl, Cyano, Methoxy oder insbesondere Chlor bedeutet.

Besonders bevorzugt sind Pyrimidine der Formel I, in denen der Substituent in 2-Stellung -N-Tₚ-X-C(=Z)-Yₒ- einen Lactamring darstellt.
- T: bedeutet eine Gruppe -CH-R^{a}-,
R^{a} steht für Wasserstoff, Halogen, Cyan oder C₁-C₃-Alkyl, besonders bevorzugt Wasserstoff
p für eine Zahl von 1 oder 4;
- X: eine Gruppe -CH-R^{a}-, -N-R^{b}-, -O- oder-S-;besonders bevorzugt -CH-R^{a}-,
R^{a} Wasserstoff oder C₁-C₃-Alkyl;
o bedeutet 0;
- Z: steht für O, S der NR^{c}, vorzugsweise für O, S, NH, NCH₃ oder NOCH₃ und besonders bevorzugt für O oder S.

Weiterhin bevorzugt sind Pyrimidine der Formel I, in denen der Substituent in 2-Stellung -N-Tₚ-X-C(=Z)-Yₒ- die folgende Bedeutung hat:
- T: bedeutet eine Gruppe -CH-R^{e}-,
R^{e} steht für Wasserstoff, Halogen, Cyan oder C₁-C₃-Alkyl, besonders bevorzugt Wasserstoff
p für eine Zahl von 1 oder 4;
- X: eine Gruppe -CH-R^{a}-, -N-R^{b}-, -O- oder-S-;besonders bevorzugt -CH-R^{a}-,
R^{a} Wasserstoff oder C₁-C₃-Alkyl;
- Y: bedeutet eine Gruppe -N-R^{b}-,
R^{b} steht für Wasserstoff oder C₁-C₃-Alkyl;
o bedeutet 1 und
- Z: steht für O, S oder NR^{c}, vorzugsweise für O oder S, NH, NCH₃ oder NOCH₃ und besonders bevorzugt für O oder S.

Weiterhin bevorzugt sind 2-substituierte Pyrimidine der Formel I wobei
- n: eine ganze Zahl von 1 bis 3 ist, wobei mindestens ein Substituent L in ortho-Stellung am Phenylring sitzt;
- L: Halogen, Cyano, Methyl, Methoxy, -C(=O)-O-A, -C(=O)-N(A')A, C(A')(=N-OA), N(A')A, N(A')-C(=O)-A,
A,A' unabhängig voneinander Wasserstoff, C₁-C₆-Alkyl, C₂-C₆-Alkenyl, C₂-C₆-Alkinyl, Phenyl, wobei die organischen Reste partiell oder vollständig halogeniert sein können oder durch C₁-C₄-Alkoxy substituiert sein können, oder A und A' zusammen mit den Atomen an die sie gebunden sind für einen fünf- bis sechsgliedrigen gesättigten Heterocyclus, enthaltend ein oder zwei Heteroatome aus der Gruppe O, N oder S stehen; wobei die aliphatischen Gruppen der Restedefinitionen von L ihrerseits partiell oder vollständig halogeniert sein können;
- R¹,R²: unabhängig voneinander C₁-C₆-Alkyl, C₂-C₆-Alkenyl, C₂-C₆-Alkinyl, C₁-C₆-Halogenalkyl, C₂-C₆-Halogenalkenyl oder C₂-C₆-Halogenalkinyl;
R² kann zusätzlich Wasserstoff bedeuten;
R¹ und R² können auch zusammen mit dem Stickstoffatom, an das sie gebunden sind, einen gesättigten oder ungesättigten fünf- oder sechsgliedrigen Ring bilden, der durch eine Ether -(-O-) oder eine weitere Amino-(-N(R^{a})- Gruppe, wobei R^{a} Wasserstoff oder C₁-C₆-Alkyl bedeutet, unterbrochen sein und/oder einen oder mehrere Substituenten aus der Gruppe Halogen, C₁-C₆-Alkyl, C₁-C₆-Halogenalkyl und Oxy-C₁-C₃-alkylenoxy enthalten kann;
- R³: Halogen, Cyano, C₁-C₄-Alkyl, C₁-C₄-Alkoxy oder C₁-C₄-Haloalkyl;
- T: bedeutet eine Gruppe -CH-R^{a}-,
R^{a} steht für Wasserstoff, Halogen, Cyan oder C₁-C₃-Alkyl, besonders bevorzugt Wasserstoff
p für eine Zahl von 1 oder 4;
- X: eine Gruppe -CH-R^{a}-, -N-R^{b}-, -O- oder -S-; besonders bevorzugt -CH-R^{a}-,
R^{a} Wasserstoff oder C₁-C₃-Alkyl;
o bedeutet 0;
- Z: steht für O, S, NH, NCH₃ oder NOCH₃ und besonders bevorzugt für O oder S.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

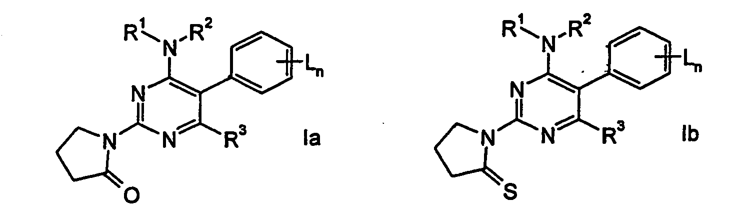

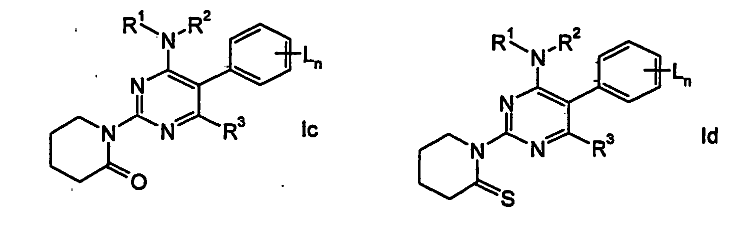

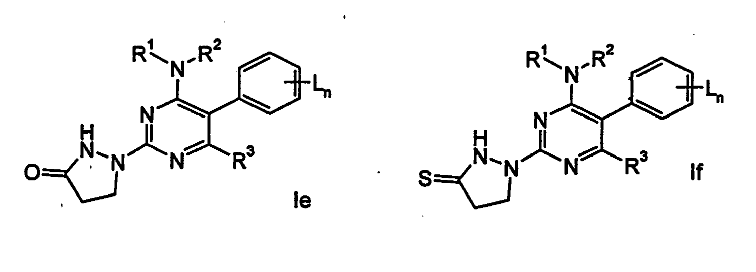

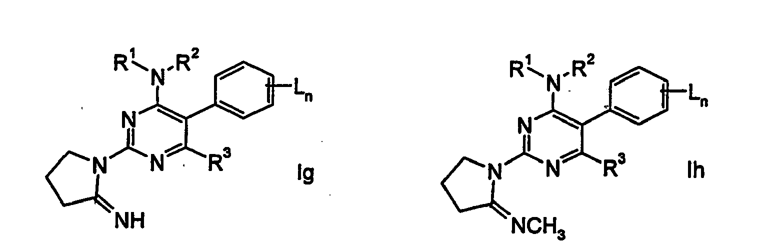

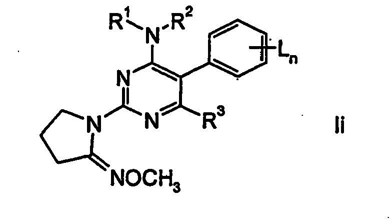

### Tabelle 1

Verbindungen der Formel Ia, Ib, Ic, Id, Ie, If, Ig, Ih und Ii, in denen Lₙ 2-Fluor,6-chlor, R³ Methyl bedeuten und R¹, R² für eine Verbindung jeweils einer Zeile der Tabelle A entspricht

### Tabelle 2

Verbindungen der Formel Ia, Ib, Ic, Id, Ie, If, Ig, Ih und Ii, in denen Lₙ 2,6-Difluor, R³ Methyl bedeuten und R¹, R² für eine Verbindung jeweils einer Zeile der Tabelle A entspricht

### Tabelle 3

Verbindungen der Formel Ia, Ib, Ic, Id, Ie, If, Ig, Ih und Ii, in denen Lₙ 2,6-Dichlor, R³ Methyl bedeuten und R¹, R² für eine Verbindung jeweils einer Zeile der Tabelle A entspricht

### Tabelle 4

Verbindungen der Formel Ia, Ib, Ic, Id, Ie, If, Ig, Ih und Ii, in denen Lₙ 2-Fluor,6-methyl, R³ Methyl bedeuten und R¹, R² für eine Verbindung jeweils einer Zeile der Tabelle A entspricht

### Tabelle 5

Verbindungen der Formel Ia, Ib, Ic, Id, Ie, If, Ig, Ih und Ii, in denen Lₙ 2,4,6-Trifluor, R³ Methyl bedeuten und R¹, R² für eine Verbindung jeweils einer Zeile der Tabelle A entspricht

### Tabelle 6

Verbindungen der Formel Ia, Ib, Ic, Id, Ie, If, Ig, Ih und Ii, in denen Lₙ 2-Methyl,4-fluor, R³ Methyl bedeuten und R¹, R² für eine Verbindung jeweils einer Zeile der Tabelle A entspricht

### Tabelle 7

Verbindungen der Formel Ia, Ib, Ic, Id, Ie, If, Ig, Ih und Ii, in denen Lₙ 2-Fluor,4-methoxycarbonyl, R³ Methyl bedeuten und R¹, R² für eine Verbindung jeweils einer Zeile der Tabelle A entspricht

### Tabelle 8

Verbindungen der Formel Ia, Ib, Ic, Id, Ie, If, Ig, Ih und Ii, in denen Lₙ 2-Fluor,4-CN, R³ Methyl bedeuten und R¹, R² für eine Verbindung jeweils einer Zeile der Tabelle A entspricht

### Tabelle 9

Verbindungen der Formel Ia, Ib, Ic, Id, Ie, If, Ig, Ih und Ii, in denen Lₙ 2,4,5-Trifluor, R³ Methyl bedeuten und R¹, R² für eine Verbindung jeweils einer Zeile der Tabelle A entspricht

### Tabelle 10

Verbindungen der Formel Ia, Ib, Ic, Id, Ie, If, Ig, Ih und Ii, in denen Lₙ 2,4-Dichlor, R³ Methyl bedeuten und R¹, R² für eine Verbindung jeweils einer Zeile der Tabelle A entspricht

### Tabelle 11

Verbindungen der Formel Ia, Ib, Ic, Id, Ie, If, Ig, Ih und Ii, in denen Lₙ 2-Chlor, R³ Methyl bedeuten und R¹, R² für eine Verbindung jeweils einer Zeile der Tabelle A entspricht

### Tabelle 12

Verbindungen der Formel Ia, Ib, Ic, Id, Ie, If, Ig, Ih und Ii, in denen Lₙ 2-Fluor, R³ Methyl bedeuten und R¹, R² für eine Verbindung jeweils einer Zeile der Tabelle A entspricht

### Tabelle 13

Verbindungen der Formel Ia, Ib, Ic, Id, Ie, If, Ig, Ih und Ii, in denen Lₙ 2,4-Difluor, R³ Methyl bedeuten und R¹, R² für eine Verbindung jeweils einer Zeile der Tabelle A entspricht

### Tabelle 14

Verbindungen der Formel Ia, Ib, Ic, Id, Ie, If, Ig, Ih und Ii, in denen Lₙ 2-Fluor-4-chlor, R³ Methyl bedeuten und R¹, R² für eine Verbindung jeweils einer Zeile der Tabelle A entspricht

### Tabelle 15

Verbindungen der Formel Ia, Ib, Ic, Id, Ie, If, Ig, Ih und Ii, in denen Lₙ 2-Chlor-4-fluor, R³ Methyl bedeuten und R¹, R² für eine Verbindung jeweils einer Zeile der Tabelle A entspricht

### Tabelle 16

Verbindungen der Formel Ia, Ib, Ic, Id, Ie, If, Ig, Ih und Ii, in denen Lₙ 2,3-Difluor, R³ Methyl bedeuten und R¹, R² für eine Verbindung jeweils einer Zeile der Tabelle A entspricht

### Tabelle 17

Verbindungen der Formel Ia, Ib, Ic, Id, Ie, If, Ig, Ih und Ii, in denen Lₙ 2,5-Difluor, R³ Methyl bedeuten und R¹, R² für eine Verbindung jeweils einer Zeile der Tabelle A entspricht

### Tabelle 18

Verbindungen der Formel Ia, Ib, Ic, Id, Ie, If, Ig, Ih und Ii, in denen Lₙ 2,3,4-Trifluor, R³ Methyl bedeuten und R¹, R² für eine Verbindung jeweils einer Zeile der Tabelle A entspricht

### Tabelle 19

Verbindungen der Formel Ia, Ib, Ic, Id, Ie, If, Ig, Ih und Ii, in denen Lₙ 2-Methyl, R³ Methyl bedeuten und R¹, R² für eine Verbindung jeweils einer Zeile der Tabelle A entspricht

### Tabelle 20

Verbindungen der Formel Ia, Ib, Ic, Id, Ie, If, Ig, Ih und Ii, in denen Lₙ 2,4-Dimethyl, R³ Methyl bedeuten und R¹, R² für eine Verbindung jeweils einer Zeile der Tabelle A entspricht

### Tabelle 21

Verbindungen der Formel Ia, Ib, Ic, Id, Ie, If, Ig, Ih und Ii, in denen Lₙ 2-Methyl-4-chlor, R³ Methyl bedeuten und R¹, R² für eine Verbindung jeweils einer Zeile der Tabelle A entspricht

### Tabelle 22

Verbindungen der Formel Ia, Ib, Ic, Id, Ie, If, Ig, Ih und Ii, in denen Lₙ 2-Fluor-4-methyl, R³ Methyl bedeuten und R¹, R² für eine Verbindung jeweils einer Zeile der Tabelle A entspricht

### Tabelle 23

Verbindungen der Formel Ia, Ib, Ic, Id, Ie, If, Ig, Ih und Ii, in denen Lₙ 2,6-Dimethyl, R³ Methyl bedeuten und R¹, R² für eine Verbindung jeweils einer Zeile der Tabelle A entspricht

### Tabelle 24

Verbindungen der Formel Ia, Ib, Ic, Id, Ie, If, Ig, Ih und Ii, in denen Lₙ 2,4,6-Trimethyl, R³ Methyl bedeuten und R¹, R² für eine Verbindung jeweils einer Zeile der Tabelle A entspricht

### Tabelle 25

Verbindungen der Formel Ia, Ib, Ic, Id, Ie, If, Ig, Ih und Ii, in denen Lₙ 2,6-Difluor-4-cyano, R³ Methyl bedeuten und R¹, R² für eine Verbindung jeweils einer Zeile der Tabelle A entspricht

### Tabelle 26

Verbindungen der Formel Ia, Ib, Ic, Id, Ie, If, Ig, Ih und Ii, in denen Lₙ 2,6-Difluor-4-methyl, R³ Methyl bedeuten und R¹, R² für eine Verbindung jeweils einer Zeile der Tabelle A entspricht

### Tabelle 27

Verbindungen der Formel Ia, Ib, Ic, Id, Ie, If, Ig, Ih und Ii, in denen Lₙ 2,6-Difluor-4-methoxycarbonyl, R³ Methyl bedeuten und R¹, R² für eine Verbindung jeweils einer Zeile der Tabelle A entspricht

### Tabelle 28

Verbindungen der Formel Ia, Ib, Ic, Id, Ie, If, Ig, Ih und Ii, in denen Lₙ 2-Chlor,4-Methoxy, R³ Methyl bedeuten und R¹, R² für eine Verbindung jeweils einer Zeile der Tabelle A entspricht

### Tabelle 29

Verbindungen der Formel Ia, Ib, Ic, Id, Ie, If, Ig, Ih und Ii, in denen Lₙ 2-Chlor,4-Methyl, R³ Methyl bedeuten und R¹, R² für eine Verbindung jeweils einer Zeile der Tabelle A entspricht

### Tabelle 30

Verbindungen der Formel Ia, Ib, Ic, Id, Ie, If, Ig, Ih und Ii, in denen Lₙ 2-Chlor,4-methoxycarbonyl, R³ Methyl bedeuten und R¹, R² für eine Verbindung jeweils einer Zeile der Tabelle A entspricht

### Tabelle 31

Verbindungen der Formel Ia, Ib, Ic, Id, Ie, If, Ig, Ih und Ii, in denen Lₙ 2-Chlor,4-Brom, R³ Methyl bedeuten und R¹, R² für eine Verbindung jeweils einer Zeile der Tabelle A entspricht

### Tabelle 32

Verbindungen der Formel Ia, Ib, Ic, Id, Ie, If, Ig, Ih und Ii, in denen Lₙ 2-Chlor,4-Cyan, R³ Methyl bedeuten und R¹, R² für eine Verbindung jeweils einer Zeile der Tabelle A entspricht

### Tabelle 33

Verbindungen der Formel Ia, Ib, Ic, Id, Ie, If, Ig, Ih und Ii, in denen Lₙ 2,6-Difluor,4-methoxy, R³ Methyl bedeuten und R¹, R² für eine Verbindung jeweils einer Zeile der Tabelle A entspricht

### Tabelle 34

Verbindungen der Formel Ia, Ib, Ic, Id, Ie, If, Ig, Ih und Ii, in denen Lₙ 2-Fluor,3-methyl, R³ Methyl bedeuten und R¹, R² für eine Verbindung jeweils einer Zeile der Tabelle A entspricht

### Tabelle 35

Verbindungen der Formel Ia, Ib, Ic, Id, Ie, If, Ig, Ih und Ii, in denen Lₙ 2,5-Dimethyl, R³ Methyl bedeuten und R¹, R² für eine Verbindung jeweils einer Zeile der Tabelle A entspricht

### Tabelle 36

Verbindungen der Formel Ia, Ib, Ic, Id, Ie, If, Ig, Ih und Ii, in denen Lₙ 2-Methyl,4-Cyan, R³ Methyl bedeuten und R¹, R² für eine Verbindung jeweils einer Zeile der Tabelle A entspricht

### Tabelle 37

Verbindungen der Formel Ia, Ib, Ic, Id, Ie, If, Ig, Ih und Ii, in denen Lₙ 2-Methyl, 4-brom, R³ Methyl bedeuten und R¹, R² für eine Verbindung jeweils einer Zeile der Tabelle A entspricht

### Tabelle 38

Verbindungen der Formel Ia, Ib, Ic, Id, Ie, If, Ig, Ih und Ii, in denen Lₙ 2-Methyl,5-fluor, R³ Methyl bedeuten und R¹, R² für eine Verbindung jeweils einer Zeile der Tabelle A entspricht

### Tabelle 39

Verbindungen der Formel Ia, Ib, Ic, Id, Ie, If, Ig, Ih und Ii, in denen Lₙ 2-Methyl,4-methoxy, R³ Methyl bedeuten und R¹, R² für eine Verbindung jeweils einer Zeile der Tabelle A entspricht

### Tabelle 40

Verbindungen der Formel Ia, Ib, Ic, Id, Ie, If, Ig, Ih und Ii, in denen Lₙ 2-Methyl,4-methoxycarbonyl, R³ Methyl bedeuten und R¹, R² für eine Verbindung jeweils einer Zeile der Tabelle A entspricht

### Tabelle 41

Verbindungen der Formel Ia, Ib, Ic, Id, Ie, If, Ig, Ih und Ii, in denen Lₙ 2,5-Dimethyl,4-brom, R³ Methyl bedeuten und R¹, R² für eine Verbindung jeweils einer Zeile der Tabelle A entspricht

### Tabelle 42

Verbindungen der Formel Ia, Ib, Ic, Id, Ie, If, Ig, Ih und Ii, in denen Lₙ 2-Fiuor,4-brom, R³ Methyl bedeuten und R¹, R² für eine Verbindung jeweils einer Zeile der Tabelle A entspricht

### Tabelle 43

Verbindungen der Formel Ia, Ib, Ic, Id, Ie, If, Ig, Ih und Ii, in denen Lₙ 2-Fluor,4-methoxy, R² Methyl bedeuten und R¹, R² für eine Verbindung jeweils einer Zeile der Tabelle A entspricht

### Tabelle 44

Verbindungen der Formel Ia, Ib, Ic, Id, Ie, If, Ig, Ih und Ii, in denen Lₙ 2-Fluor,5-methyl, R³ Methyl bedeuten und R¹, R² für eine Verbindung jeweils einer Zeile der Tabelle A entspricht

### Tabelle 45

Verbindungen der Formel Ia, Ib, Ic, Id, Ie, If, Ig, Ih und Ii, in denen Lₙ Pentafluor, R³ Methyl bedeuten und R¹, R² für eine Verbindung jeweils einer Zeile der Tabelle A entspricht

### Tabelle 46

Verbindungen der Formel Ia, Ib, Ic, Id, Ie, If, Ig, Ih und Ii, in denen Lₙ 2-Fluor,6-chlor, R³ Chlor bedeuten und R¹, R² für eine Verbindung jeweils einer Zeile der Tabelle A entspricht

### Tabelle 47

Verbindungen der Formel Ia, Ib, Ic, Id, Ie, If, Ig, Ih und Ii, in denen Lₙ 2,6-Difluor, R³ Chlor bedeuten und R¹, R² für eine Verbindung jeweils einer Zeile der Tabelle A entspricht

### Tabelle 48

Verbindungen der Formel Ia, Ib, Ic, Id, Ie, If, Ig, Ih und Ii, in denen Lₙ 2,6-Dichlor, R³ Chlor bedeuten und R¹, R² für eine Verbindung jeweils einer Zeile der Tabelle A entspricht

### Tabelle 49

Verbindungen der Formel Ia, Ib, Ic, Id, Ie, If, Ig, Ih und Ii, in denen Lₙ 2-Fluor,6-methyl, R³ Chlor bedeuten und R¹, R² für eine Verbindung jeweils einer Zeile der Tabelle A entspricht

### Tabelle 50

Verbindungen der Formel Ia, Ib, Ic, Id, Ie, If, Ig, Ih und Ii, in denen Lₙ 2,4,6-Trifluor, R³ Chlor bedeuten und R¹, R² für eine Verbindung jeweils einer Zeile der Tabelle A entspricht

### Tabelle 51

Verbindungen der Formel Ia, Ib, Ic, Id, Ie, If, Ig, Ih und Ii, in denen Lₙ 2-Methyl,4-fluor, R³ Chlor bedeuten und R¹, R² für eine Verbindung jeweils einer Zeile der Tabelle A entspricht

### Tabelle 52

Verbindungen der Formel Ia, Ib, Ic, Id, Ie, If, Ig, Ih und Ii, in denen Lₙ 2-Fluor,4-methoxycarbonyl, R³ Chlor bedeuten und R¹, R² für eine Verbindung jeweils einer Zeile der Tabelle A entspricht

### Tabelle 53

Verbindungen der Formel Ia, Ib, Ic, Id, Ie, If, Ig, Ih und Ii, in denen Lₙ 2-Fluor,4-CN, R³ Chlor bedeuten und R¹, R² für eine Verbindung jeweils einer Zeile der Tabelle A entspricht

### Tabelle 54

Verbindungen der Formel Ia, Ib, Ic, Id, Ie, If, Ig, Ih und Ii, in denen Lₙ 2,4,5-Trifluor, R³ Chlor bedeuten und R¹, R² für eine Verbindung jeweils einer Zeile der Tabelle A entspricht

### Tabelle 55

Verbindungen der Formel Ia, Ib, Ic, Id, Ie, If, Ig, Ih und Ii, in denen Lₙ 2,4-Dichlor, R³ Chlor bedeuten und R¹, R² für eine Verbindung jeweils einer Zeile der Tabelle A entspricht

### Tabelle 56

Verbindungen der Formel Ia, Ib, Ic, Id, Ie, If, Ig, Ih und Ii, in denen Lₙ 2-Chlor, R³ Chlor bedeuten und R¹, R² für eine Verbindung jeweils einer Zeile der Tabelle A entspricht

### Tabelle 57

Verbindungen der Formel Ia, Ib, Ic, Id, Ie, If, Ig, Ih und Ii, in denen Lₙ 2-Fluor, R³ Chlor bedeuten und R¹, R² für eine Verbindung jeweils einer Zeile der Tabelle A entspricht

### Tabelle 58

Verbindungen der Formel Ia, Ib, Ic, Id, Ie, If, Ig, Ih und Ii, in denen Lₙ 2,4-Difluor, R³ Chlor bedeuten und R¹, R² für eine Verbindung jeweils einer Zeile der Tabelle A entspricht

### Tabelle 59

Verbindungen der Formel Ia, Ib, Ic, Id, Ie, If, Ig, Ih und Ii, in denen Lₙ 2-Fluor-4-chlor, R³ Chlor bedeuten und R¹, R² für eine Verbindung jeweils einer Zeile der Tabelle A entspricht

### Tabelle 60

Verbindungen der Formel Ia, Ib, Ic, Id, Ie, If, Ig, Ih und Ii, in denen Lₙ 2-Chlor-4-fluor, R³ Chlor bedeuten und R¹, R² für eine Verbindung jeweils einer Zeile der Tabelle A entspricht

### Tabelle 61

Verbindungen der Formel Ia, Ib, Ic, Id, Ie, If, Ig, Ih und Ii, in denen Lₙ 2,3-Difluor, R³ Chlor bedeuten und R¹, R² für eine Verbindung jeweils einer Zeile der Tabelle A entspricht

### Tabelle 62

Verbindungen der Formel Ia, Ib, Ic, Id, Ie, If, Ig, Ih und Ii, in denen Lₙ 2,5-Difluor, R³ Chlor bedeuten und R¹, R² für eine Verbindung jeweils einer Zeile der Tabelle A entspricht

### Tabelle 63

Verbindungen der Formel Ia, Ib, Ic, Id, Ie, If, Ig, Ih und Ii, in denen Lₙ 2,3,4-Trifluor, R³ Chlor bedeuten und R¹, R² für eine Verbindung jeweils einer Zeile der Tabelle A entspricht

### Tabelle 64

Verbindungen der Formel Ia, Ib, Ic, Id, Ie, If, Ig, Ih und Ii, in denen Lₙ 2-Methyl, R³ Chlor bedeuten und R¹, R² für eine Verbindung jeweils einer Zeile der Tabelle A entspricht

### Tabelle 65

Verbindungen der Formel Ia, Ib, Ic, Id, Ie, If, Ig, Ih und Ii, in denen Lₙ 2,4-Dimethyl, R³ Chlor bedeuten und R¹, R² für eine Verbindung jeweils einer Zeile der Tabelle A entspricht

### Tabelle 66

Verbindungen der Formel Ia, Ib, Ic, Id, Ie, If, Ig, Ih und Ii, in denen Lₙ 2-Methyl-4-chlor, R³ Chlor bedeuten und R¹, R² für eine Verbindung jeweils einer Zeile der Tabelle A entspricht

### Tabelle 67

Verbindungen der Formel Ia, Ib, Ic, Id, Ie, If, Ig, Ih und Ii, in denen Lₙ 2-Fluor-4-methyl, R² Chlor bedeuten und R¹, R² für eine Verbindung jeweils einer Zeile der Tabelle A entspricht "

### Tabelle 68

Verbindungen der Formel Ia, Ib, Ic, Id, Ie, If, Ig, Ih und Ii, in denen Lₙ 2,6-Dimethyl, R³ Chlor bedeuten und R¹, R² für eine Verbindung jeweils einer Zeile der Tabelle A entspricht

### Tabelle 69

Verbindungen der Formel Ia, Ib, Ic, Id, Ie, If, Ig, Ih und Ii, in denen Lₙ 2,4,6-Trimethyl, R³ Chlor bedeuten und R¹, R² für eine Verbindung jeweils einer Zeile der Tabelle A entspricht

### Tabelle 70

Verbindungen der Formel Ia, Ib, Ic, Id, Ie, If, Ig, Ih und Ii, in denen Lₙ 2,6-Difluor-4-cyano, R³ Chlor bedeuten und R¹, R² für eine Verbindung jeweils einer Zeile der Tabelle A entspricht

### Tabelle 71

Verbindungen der Formel Ia, Ib, Ic, Id, Ie, If, Ig, Ih und Ii, in denen Lₙ 2,6-Difluor-4-methyl, R³ Chlor bedeuten und R¹, R² für eine Verbindung jeweils einer Zeile der Tabelle A entspricht

### Tabelle 72

Verbindungen der Formel Ia, Ib, Ic, Id, Ie, If, Ig, Ih und Ii, in denen Lₙ 2,6-Difluor-4-methoxycarbonyl, R³ Chlor bedeuten und R¹, R² für eine Verbindung jeweils einer Zeile der Tabelle A entspricht

### Tabelle 73

Verbindungen der Formel Ia, Ib, Ic, Id, Ie, If, Ig, Ih und Ii, in denen Lₙ 2-Chlor,4-Methoxy, R³ Chlor bedeuten und R¹, R² für eine Verbindung jeweils einer Zeile der Tabelle A entspricht

### Tabelle 74

Verbindungen der Formel Ia, Ib, Ic, Id, Ie, If, Ig, Ih und Ii, in denen Lₙ 2-Chlor,4-Methyl, R³ Chlor bedeuten und R¹, R² für eine Verbindung jeweils einer Zeile der Tabelle A entspricht

### Tabelle 75

Verbindungen der Formel Ia, Ib, Ic, Id, Ie, If, Ig, Ih und Ii, in denen Lₙ 2-Chlor,4-methoxycarbonyl, R³ Chlor bedeuten und R¹, R² für eine Verbindung jeweils einer Zeile der Tabelle A entspricht

### Tabelle 76

Verbindungen der Formel Ia, Ib, Ic, Id, Ie, If, Ig, Ih und Ii, in denen Lₙ 2-Chlor,4-Brom, R³ Chlor bedeuten und R¹, R² für eine Verbindung jeweils einer Zeile der Tabelle A entspricht

### Tabelle 77

Verbindungen der Formel Ia, Ib, Ic, Id, Ie, If, Ig, Ih und Ii, in denen Lₙ 2-Chlor,4-Cyan, R³ Chlor bedeuten und R¹, R² für eine Verbindung jeweils einer Zeile der Tabelle A entspricht

### Tabelle 78

Verbindungen der Formel Ia, Ib, Ic, Id, Ie, If, Ig, Ih und Ii, in denen Lₙ 2,6-Difluor,4-methoxy, R³ Chlor bedeuten und R¹, R² für eine Verbindung jeweils einer Zeile der Tabelle A entspricht

### Tabelle 79

Verbindungen der Formel Ia, Ib, Ic, Id, Ie, If, Ig, Ih und Ii, in denen Lₙ 2-Fluor,3-methyl, R³ Chlor bedeuten und R¹, R² für eine Verbindung jeweils einer Zeile der Tabelle A entspricht

### Tabelle 80

Verbindungen der Formel Ia, Ib, Ic, Id, Ie, If, Ig, Ih und Ii, in denen Lₙ 2,5-Dimethyl, R³ Chlor bedeuten und R¹, R² für eine Verbindung jeweils einer Zeile der Tabelle A entspricht

### Tabelle 81

Verbindungen der Formel Ia, Ib, Ic, Id, Ie, If, Ig, Ih und Ii, in denen Lₙ 2-Methyl,4-cyan, R³ Chlor bedeuten und R¹, R² für eine Verbindung jeweils einer Zeile der Tabelle A entspricht

### Tabelle 82

Verbindungen der Formel Ia, Ib, Ic, Id, Ie, If, Ig, Ih und Ii, in denen Lₙ 2-Methyl,4-brom, R³ Chlor bedeuten und R¹, R² für eine Verbindung jeweils einer Zeile der Tabelle A entspricht

### Tabelle 83

Verbindungen der Formel Ia, Ib, Ic, Id, Ie, If, Ig, Ih und Ii, in denen Lₙ 2-Methyl,5-fluor, R³ Chlor bedeuten und R¹, R² für eine Verbindung jeweils einer Zeile der Tabelle A entspricht

### Tabelle 84

Verbindungen der Formel Ia, Ib, Ic, Id, Ie, If, Ig, Ih und Ii, in denen Lₙ 2-Methyl,4-methoxy, R³ Chlor bedeuten und R¹, R² für eine Verbindung jeweils einer Zeile der Tabelle A entspricht

### Tabelle 85

Verbindungen der Formel Ia, Ib, Ic, Id, Ie, If, Ig, Ih und Ii, in denen Lₙ 2-Methyl,4-methoxycarbonyl, R³ Chlor bedeuten und R¹, R² für eine Verbindung jeweils einer Zeile der Tabelle A entspricht

### Tabelle 86

Verbindungen der Formel Ia, Ib, Ic, Id, Ie, If, Ig, Ih und Ii, in denen Lₙ 2,5-Dimethyl,4-brom, R³ Chlor bedeuten und R¹, R² für eine Verbindung jeweils einer Zeile der Tabelle A entspricht

### Tabelle 87

Verbindungen der Formel Ia, Ib, Ic, Id, Ie, If, Ig, Ih und Ii, in denen Lₙ 2-Fluor,4-brom, R³ Chlor bedeuten und R¹, R² für eine Verbindung jeweils einer Zeile der Tabelle A entspricht

### Tabelle 88

Verbindungen der Formel Ia, Ib, Ic, Id, Ie, If, Ig, Ih und Ii, in denen Lₙ 2-Fluor,4-methoxy, R³ Chlor bedeuten und R¹, R² für eine Verbindung jeweils einer Zeile der Tabelle A entspricht

### Tabelle 89

Verbindungen der Formel Ia, Ib, Ic, Id, Ie, If, Ig, Ih und Ii, in denen Lₙ 2-Fluor,5-methyl, R³ Chlor bedeuten und R¹, R² für eine Verbindung jeweils einer Zeile der Tabelle A entspricht

### Tabelle 90

Verbindungen der Formel Ia, Ib, Ic, Id, Ie, If, Ig, Ih und Ii, in denen Lₙ Pentafluor, R³ Chlor bedeuten und R¹, R² für eine Verbindung jeweils einer Zeile der Tabelle A entspricht

### Tabelle 91

Verbindungen der Formel Ia, Ib, Ic, Id, Ie, If, Ig, Ih und Ii, in denen Lₙ 2-Fluor,6-chlor, R³ Methoxy bedeuten und R¹, R² für eine Verbindung jeweils einer Zeile der Tabelle A entspricht

### Tabelle 92

Verbindungen der Formel Ia, Ib, Ic, Id, Ie, If, Ig, Ih und Ii, in denen Lₙ 2,6-Difluor, R³ Methoxy bedeuten und R¹, R² für eine Verbindung jeweils einer Zeile der Tabelle A entspricht

### Tabelle 93

Verbindungen der Formel Ia, Ib, Ic, Id, Ie, If, Ig, Ih und Ii, in denen Lₙ 2,6-Dichlor, R³ Methoxy bedeuten und R¹, R² für eine Verbindung jeweils einer Zeile der Tabelle A entspricht

### Tabelle 94

Verbindungen der Formel Ia, Ib, Ic, Id, Ie, If, Ig, Ih und Ii, in denen Lₙ 2-Fluor,6-methyl, R³ Methoxy bedeuten und R¹, R² für eine Verbindung jeweils einer Zeile der Tabelle A entspricht

### Tabelle 95

Verbindungen der Formel Ia, Ib, Ic, Id, Ie, If, Ig, Ih und Ii, in denen Lₙ 2,4,6-Trifluor, R³ Methoxy bedeuten und R¹, R² für eine Verbindung jeweils einer Zeile der Tabelle A entspricht

### Tabelle 96

Verbindungen der Formel Ia, Ib, Ic, Id, Ie, If, Ig, Ih und Ii, in denen Lₙ 2-Methyl,4-fluor, R³ Methoxy bedeuten und R¹, R² für eine Verbindung jeweils einer Zeile der Tabelle A entspricht

### Tabelle 97

Verbindungen der Formel Ia, Ib, Ic, Id, Ie, If, Ig, Ih und Ii, in denen Lₙ 2-Fluor,4-methoxycarbonyl, R³ Methoxy bedeuten und R¹, R² für eine Verbindung jeweils einer Zeile der Tabelle A entspricht

### Tabelle 98

Verbindungen der Formel Ia, Ib, Ic, Id, Ie, If, Ig, Ih und Ii, in denen Lₙ 2-Fluor,4-CN, R³ Methoxy bedeuten und R¹, R² für eine Verbindung jeweils einer Zeile der Tabelle A entspricht

### Tabelle 99

Verbindungen der Formel Ia, Ib, Ic, Id, Ie, If, Ig, Ih und Ii, in denen Lₙ 2,4,5-Trifluor, R³ Methoxy bedeuten und R¹, R² für eine Verbindung jeweils einer Zeile der Tabelle A entspricht

### Tabelle 100

Verbindungen der Formel Ia, Ib, Ic, Id, Ie, If, Ig, Ih und Ii, in denen Lₙ 2,4-Dichlor, R³ Methoxy bedeuten und R¹, R² für eine Verbindung jeweils einer Zeile der Tabelle A entspricht

### Tabelle 101

Verbindungen der Formel Ia, Ib, Ic, Id, Ie, If, Ig, Ih und Ii, in denen Lₙ 2-Chlor, R³ Methoxy bedeuten und R¹, R² für eine Verbindung jeweils einer Zeile der Tabelle A entspricht

### Tabelle 102

Verbindungen der Formel Ia, Ib, Ic, Id, Ie, If, Ig, Ih und Ii, in denen Lₙ 2-Fluor, R³ Methoxy bedeuten und R¹, R² für eine Verbindung jeweils einer Zeile der Tabelle A entspricht

### Tabelle 103

Verbindungen der Formel Ia, Ib, Ic, Id, Ie, If, Ig, Ih und Ii, in denen Lₙ 2,4-Difluor, R³ Methoxy bedeuten und R¹, R² für eine Verbindung jeweils einer Zeile der Tabelle A entspricht

### Tabelle 104

Verbindungen der Formel Ia, Ib, Ic, Id, Ie, If, Ig, Ih und Ii, in denen Lₙ 2-Fluor-4-chlor, R³ Methoxy bedeuten und R¹, R² für eine Verbindung jeweils einer Zeile der Tabelle A entspricht

### Tabelle 105

Verbindungen der Formel Ia, Ib, Ic, Id, Ie, If, Ig, Ih und Ii, in denen Lₙ 2-Chlor-4-fluor, R³ Methoxy bedeuten und R¹, R² für eine Verbindung jeweils einer Zeile der Tabelle A entspricht

### Tabelle 106

Verbindungen der Formel Ia, Ib, Ic, Id, Ie, If, Ig, Ih und Ii, in denen Lₙ 2,3-Difluor, R³ Methoxy bedeuten und R¹, R² für eine Verbindung jeweils einer Zeile der Tabelle A entspricht

### Tabelle 107

Verbindungen der Formel Ia, Ib, Ic, Id, Ie, If, Ig, Ih und Ii, in denen Lₙ 2,5-Difluor, R³ Methoxy bedeuten und R¹, R² für eine Verbindung jeweils einer Zeile der Tabelle A entspricht

### Tabelle 108

Verbindungen der Formel Ia, Ib, Ic, Id, Ie, If, Ig, Ih und Ii, in denen Lₙ 2,3,4-Trifluor, R³ Methoxy bedeuten und R¹, R² für eine Verbindung jeweils einer Zeile der Tabelle A entspricht

### Tabelle 109

Verbindungen der Formel Ia, Ib, Ic, Id, Ie, If, Ig, Ih und Ii, in denen Lₙ 2-Methyl, R³ Methoxy bedeuten und R¹, R² für eine Verbindung jeweils einer Zeile der Tabelle A entspricht

### Tabelle 110

Verbindungen der Formel Ia, Ib, Ic, Id, Ie, If, Ig, Ih und Ii, in denen Lₙ 2,4-Dimethyl, R³ Methoxy bedeuten und R¹, R² für eine Verbindung jeweils einer Zeile der Tabelle A entspricht

### Tabelle 111

Verbindungen der Formel Ia, Ib, Ic, Id, Ie, If, Ig, Ih und Ii, in denen Lₙ 2-Methyl-4-chlor, R³ Methoxy bedeuten und R¹, R² für eine Verbindung jeweils einer Zeile der Tabelle A entspricht

### Tabelle 112

Verbindungen der Formel Ia, Ib, Ic, Id, Ie, If, Ig, Ih und Ii, in denen Lₙ 2-Fluor-4-methyl, R³ Methoxy bedeuten und R¹, R² für eine Verbindung jeweils einer Zeile der Tabelle A entspricht

### Tabelle 113

Verbindungen der Formel Ia, Ib, Ic, Id, Ie, If, Ig, Ih und Ii, in denen Lₙ 2,6-Dimethyl, R³ Methoxy bedeuten und R¹, R² für eine Verbindung jeweils einer Zeile der Tabelle A entspricht

### Tabelle 114

Verbindungen der Formel Ia, Ib, Ic, Id, Ie, If, Ig, Ih und Ii, in denen Lₙ 2,4,6-Trimethyl, R³ Methoxy bedeuten und R¹, R² für eine Verbindung jeweils einer Zeile der Tabelle A entspricht

### Tabelle 115

Verbindungen der Formel Ia, Ib, Ic, Id, Ie, If, Ig, Ih und Ii, in denen Lₙ 2,6-Difluor-4-cyano, R³ Methoxy bedeuten und R¹, R² für eine Verbindung jeweils einer Zeile der Tabelle A entspricht

### Tabelle 116

Verbindungen der Formel Ia, Ib, Ic, Id, Ie, If, Ig, Ih und Ii, in denen Lₙ 2,6-Difluor-4-methyl, R³ Methoxy bedeuten und R¹, R² für eine Verbindung jeweils einer Zeile der Tabelle A entspricht

### Tabelle 117

Verbindungen der Formel Ia, Ib, Ic, Id, Ie, If, Ig, Ih und Ii, in denen Lₙ 2,6-Difluor-4-Methoxycarbonyl, R³ Methoxy bedeuten und R¹, R² für eine Verbindung jeweils einer Zeile der Tabelle A entspricht

### Tabelle 118

Verbindungen der Formel Ia, Ib, Ic, Id, Ie, If, Ig, Ih und Ii, in denen Lₙ 2-Chlor,4-Methoxy, R³ Methoxy bedeuten und R¹, R² für eine Verbindung jeweils einer Zeile der Tabelle A entspricht

### Tabelle 119

Verbindungen der Formel Ia, Ib, Ic, Id, Ie, If, Ig, Ih und Ii, in denen Lₙ 2-Chlor,4-Methyl, R³ Methoxy bedeuten und R¹, R² für eine Verbindung jeweils einer Zeile der Tabelle A entspricht

### Tabelle 120

Verbindungen der Formel Ia, Ib, Ic, Id, Ie, If, Ig, Ih und Ii, in denen Lₙ 2-Chlor,4-methoxycarbonyl, R³ Methoxy bedeuten und R¹, R² für eine Verbindung jeweils einer Zeile der Tabelle A entspricht

### Tabelle 121

Verbindungen der Formel Ia, Ib, Ic, Id, Ie, If, Ig, Ih und Ii, in denen Lₙ 2-Chlor,4-Methoxy, R³ Methoxy bedeuten und R¹, R² für eine Verbindung jeweils einer Zeile der Tabelle A entspricht

### Tabelle 122

Verbindungen der Formel Ia, Ib, Ic, Id, Ie, If, Ig, Ih und Ii, in denen Lₙ 2-Chlor,4-Cyan, R³ Methoxy bedeuten und R¹, R² für eine Verbindung jeweils einer Zeile der Tabelle A entspricht

### Tabelle 123

Verbindungen der Formel Ia, Ib, Ic, Id, Ie, If, Ig, Ih und Ii, in denen Lₙ 2,6-Difluor,4-methoxy, R³ Methoxy bedeuten und R¹, R² für eine Verbindung jeweils einer Zeile der Tabelle A entspricht

### Tabelle 124

Verbindungen der Formel Ia, Ib, Ic, Id, Ie, If, Ig, Ih und Ii, in denen Lₙ 2-Fluor,3-methyl, R³ Methoxy bedeuten und R¹, R² für eine Verbindung jeweils einer Zeile der Tabelle A entspricht

### Tabelle 125

Verbindungen der Formel Ia, Ib, Ic, Id, Ie, If, Ig, Ih und Ii, in denen Lₙ 2,5-Dimethyl, R³ Methoxy bedeuten und R¹, R² für eine Verbindung jeweils einer Zeile der Tabelle A entspricht

### Tabelle 126

Verbindungen der Formel Ia, Ib, Ic, Id, Ie, If, Ig, Ih und Ii, in denen Lₙ 2-Methyl,4-Cyan, R³ Methoxy bedeuten und R¹, R² für eine Verbindung jeweils einer Zeile der Tabelle A entspricht

### Tabelle 127

Verbindungen der Formel Ia, Ib, Ic, Id, Ie, If, Ig, Ih und Ii, in denen Lₙ 2-Methyl,4-Brom, R³ Methoxy bedeuten und R¹, R² für eine Verbindung jeweils einer Zeile der Tabelle A entspricht

### Tabelle 128

Verbindungen der Formel Ia, Ib, Ic, Id, Ie, If, Ig, Ih und Ii, in denen Lₙ 2-Methyl,5-fluor, R³ Methoxy bedeuten und R¹, R² für eine Verbindung jeweils einer Zeile der Tabelle A entspricht

### Tabelle 129

Verbindungen der Formel Ia, Ib, Ic, Id, Ie, If, Ig, Ih und Ii, in denen Lₙ 2-Methyl,4-methoxy, R³ Methoxy bedeuten und R¹, R² für eine Verbindung jeweils einer Zeile der Tabelle A entspricht

### Tabelle 130

Verbindungen der Formel Ia, Ib, Ic, Id, Ie, If, Ig, Ih und Ii, in denen Lₙ 2-Methyl,4-methoxycarbonyl, R³ Methoxy bedeuten und R¹, R² für eine Verbindung jeweils einer Zeile der Tabelle A entspricht

### Tabelle 131

Verbindungen der Formel Ia, Ib, Ic, Id, Ie, If, Ig, Ih und Ii, in denen Lₙ 2,5-Dimethyl,4-brom, R³ Methoxy bedeuten und R¹, R² für eine Verbindung jeweils einer Zeile der Tabelle A entspricht

### Tabelle 132

Verbindungen der Formel Ia, Ib, Ic, Id, Ie, If, Ig, Ih und Ii, in denen Lₙ 2-Fluor,4-brom, R³ Methoxy bedeuten und R¹, R² für eine Verbindung jeweils einer Zeile der Tabelle A entspricht

### Tabelle 133

Verbindungen der Formel Ia, Ib, Ic, Id, Ie, If, Ig, Ih und Ii, in denen Lₙ 2-Fluor,4-methoxy, R³ Methoxy bedeuten und R¹, R² für eine Verbindung jeweils einer Zeile der Tabelle A entspricht

### Tabelle 134

Verbindungen der Formel Ia, Ib, Ic, Id, Ie, If, Ig, Ih und Ii, in denen Lₙ 2-Fluor,5-methyl, R³ Methoxy bedeuten und R¹, R² für eine Verbindung jeweils einer Zeile der Tabelle A entspricht

### Tabelle 135

Verbindungen der Formel Ia, Ib, Ic, Id, Ie, If, Ig, Ih und Ii, in denen Lₙ Pentafluor, R³ Methoxy bedeuten und R¹, R² für eine Verbindung jeweils einer Zeile der Tabelle A entspricht

### Tabelle 136

Verbindungen der Formel Ia, Ib, Ic, Id, Ie, If, Ig, Ih und Ii, in denen Lₙ 2-Fluor,6-chlor, R³ Cyano bedeuten und R¹, R² für eine Verbindung jeweils einer Zeile der Tabelle A entspricht

### Tabelle 137

Verbindungen der Formel Ia, Ib, Ic, Id, Ie, If, Ig, Ih und Ii, in denen Lₙ 2,6-Difluor, R³ Cyano bedeuten und R¹, R² für eine Verbindung jeweils einer Zeile der Tabelle A entspricht

### Tabelle 138

Verbindungen der Formel Ia, Ib, Ic, Id, Ie, If, Ig, Ih und Ii, in denen Lₙ 2,6-Dichlor, R³ Cyano bedeuten und R¹, R² für eine Verbindung jeweils einer Zeile der Tabelle A entspricht

### Tabelle 139

Verbindungen der Formel Ia, Ib, Ic, Id, Ie, If, Ig, Ih und Ii, in denen Lₙ 2-Fluor,6-methyl, R³ Cyano bedeuten und R¹, R² für eine Verbindung jeweils einer Zeile der Tabelle A entspricht

### Tabelle 140

Verbindungen der Formel Ia, Ib, Ic, Id, Ie, If, Ig, Ih und Ii, in denen Lₙ 2,4,6-Trifluor, R³ Cyano bedeuten und R¹, R² für eine Verbindung jeweils einer Zeile der Tabelle A entspricht

### Tabelle 141

Verbindungen der Formel Ia, Ib, Ic, Id, Ie, If, Ig, Ih und Ii, in denen Lₙ 2-Methyl,4-fluor, R³ Cyano bedeuten und R¹, R² für eine Verbindung jeweils einer Zeile der Tabelle A entspricht

### Tabelle 142

Verbindungen der Formel Ia, Ib, Ic, Id, Ie, If, Ig, Ih und Ii, in denen Lₙ 2-Fluor,4-methoxycarbonyl, R³ Cyano bedeuten und R¹, R² für eine Verbindung jeweils einer Zeile der Tabelle A entspricht

### Tabelle 143

Verbindungen der Formel Ia, Ib, Ic, Id, Ie, If, Ig, Ih und Ii, in denen Lₙ 2-Fluor,4-CN, R³ Cyano bedeuten und R¹, R² für eine Verbindung jeweils einer Zeile der Tabelle A entspricht

### Tabelle 144

Verbindungen der Formel Ia, Ib, Ic, Id, Ie, If, Ig, Ih und Ii, in denen Lₙ 2,4,5-Trifluor, R³ Cyano bedeuten und R¹, R² für eine Verbindung jeweils einer Zeile der Tabelle A entspricht

### Tabelle 145

Verbindungen der Formel Ia, Ib, Ic, Id, Ie, If, Ig, Ih und Ii, in denen Lₙ 2,4-Dichlor, R³ Cyano bedeuten und R¹, R² für eine Verbindung jeweils einer Zeile der Tabelle A entspricht

### Tabelle 146

Verbindungen der Formel Ia, Ib, Ic, Id, Ie, If, Ig, Ih und Ii, in denen Lₙ 2-Chlor, R³ Cyano bedeuten und R¹, R² für eine Verbindung jeweils einer Zeile der Tabelle A entspricht

### Tabelle 147

Verbindungen der Formel Ia, Ib, Ic, Id, Ie, If, Ig, Ih und Ii, in denen Lₙ 2-Fluor, R³ Cyano bedeuten und R¹, R² für eine Verbindung jeweils einer Zeile der Tabelle A entspricht

### Tabelle 148 "

Verbindungen der Formel Ia, Ib, Ic, Id, Ie, If, Ig, Ih und Ii, in denen Lₙ 2,4-Difluor, R³ Cyano bedeuten und R¹, R² für eine Verbindung jeweils einer Zeile der Tabelle A entspricht

### Tabelle 149

Verbindungen der Formel Ia, Ib, Ic, Id, Ie, If, Ig, Ih und Ii, in denen Lₙ 2-Fluor-4-chlor, R³ Cyano bedeuten und R¹, R² für eine Verbindung jeweils einer Zeile der Tabelle A entspricht

### Tabelle 150

Verbindungen der Formel Ia, Ib, Ic, Id, Ie, If, Ig, Ih und Ii, in denen Lₙ 2-Chlor-4-fluor, R³ Cyano bedeuten und R¹, R² für eine Verbindung jeweils einer Zeile der Tabelle A entspricht

### Tabelle 151

Verbindungen der Formel Ia, Ib, Ic, Id, Ie, If, Ig, Ih und Ii, in denen Lₙ 2,3-Difluor, R³ Cyano bedeuten und R¹, R² für eine Verbindung jeweils einer Zeile der Tabelle A entspricht

### Tabelle 152

Verbindungen der Formel Ia, Ib, Ic, Id, Ie, If, Ig, Ih und Ii, in denen Lₙ 2,5-Difluor, R³ Cyano bedeuten und R¹, R² für eine Verbindung jeweils einer Zeile der Tabelle A entspricht

### Tabelle 153

Verbindungen der Formel Ia, Ib, Ic, Id, Ie, If, Ig, Ih und Ii, in denen Lₙ 2,3,4-Trifluor, R³ Cyano bedeuten und R¹, R² für eine Verbindung jeweils einer Zeile der Tabelle A entspricht

### Tabelle 154

Verbindungen der Formel Ia, Ib, Ic, Id, Ie, If, Ig, Ih und Ii, in denen Lₙ 2-Methyl, R³ Cyano bedeuten und R¹, R² für eine Verbindung jeweils einer Zeile der Tabelle A entspricht

### Tabelle 155

Verbindungen der Formel Ia, Ib, Ic, Id, Ie, If, Ig, Ih und Ii, in denen Lₙ 2,4-Dimethyl, R³ Cyano bedeuten und R¹, R² für eine Verbindung jeweils einer Zeile der Tabelle A entspricht

### Tabelle 156

Verbindungen der Formel Ia, Ib, Ic, Id, Ie, If, Ig, Ih und Ii, in denen Lₙ 2-Methyl-4-chlor, R³ Cyano bedeuten und R¹, R² für eine Verbindung jeweils einer Zeile der Tabelle A entspricht

### Tabelle 157

Verbindungen der Formel Ia, Ib, Ic, Id, Ie, If, Ig, Ih und Ii, in denen Lₙ 2-Fluor-4-methyl, R³ Cyano bedeuten und R¹, R² für eine Verbindung jeweils einer Zeile der Tabelle A entspricht

### Tabelle 158

Verbindungen der Formel Ia, Ib, Ic, Id, Ie, If, Ig, Ih und Ii, in denen Lₙ 2,6-Dimethyl, R³ Cyano bedeuten und R¹, R² für eine Verbindung jeweils einer Zeile der Tabelle A entspricht

### Tabelle 159

Verbindungen der Formel Ia, Ib, Ic, Id, Ie, If, Ig, Ih und Ii, in denen Lₙ 2,4,6-Trimethyl, R³ Cyano bedeuten und R¹, R² für eine Verbindung jeweils einer Zeile der Tabelle A entspricht

### Tabelle 160

Verbindungen der Formel Ia, Ib, Ic, Id, Ie, If, Ig, Ih und Ii, in denen Lₙ 2,6-Difluor-4-cyano, R³ Cyano bedeuten und R¹, R² für eine Verbindung jeweils einer Zeile der Tabelle A entspricht

### Tabelle 161

Verbindungen der Formel Ia, Ib, Ic, Id, Ie, If, Ig, Ih und Ii, in denen Lₙ 2,6-Difluor-4-methyl, R³ Cyano bedeuten und R¹, R² für eine Verbindung jeweils einer Zeile der Tabelle A entspricht

### Tabelle 162

Verbindungen der Formel Ia, Ib, Ic, Id, Ie, If, Ig, Ih und Ii, in denen Lₙ 2,6-Difluor-4-methoxycarbonyl, R³ Cyano bedeuten und R¹, R² für eine Verbindung jeweils einer Zeile der Tabelle A entspricht

### Tabelle 163

Verbindungen der Formel Ia, Ib, Ic, Id, Ie, If, Ig, Ih und Ii, in denen Lₙ 2-Chlor,4-Methoxy, R³ Cyano bedeuten und R¹, R² für eine Verbindung jeweils einer Zeile der Tabelle A entspricht

### Tabelle 164

Verbindungen der Formel Ia, Ib, Ic, Id, Ie, If, Ig, Ih und Ii, in denen Lₙ 2-Chlor,4-Methyl, R³ Cyano bedeuten und R¹, R² für eine Verbindung jeweils einer Zeile der Tabelle A entspricht

### Tabelle 165

Verbindungen der Formel Ia, Ib, Ic, Id, Ie, If, Ig, Ih und Ii, in denen Lₙ 2-Chlor,4-methoxycarbonyl, R³ Cyano bedeuten und R¹, R² für eine Verbindung jeweils einer Zeile der Tabelle A entspricht

### Tabelle 166

Verbindungen der Formel Ia, Ib, Ic, Id, Ie, If, Ig, Ih und Ii, in denen Lₙ 2-Chlor,4-Brom, R³ Cyano bedeuten und R¹, R² für eine Verbindung jeweils einer Zeile der Tabelle A entspricht

### Tabelle 167

Verbindungen der Formel Ia, Ib, Ic, Id, Ie, If, Ig, Ih und Ii, in denen Lₙ 2-Chlor,4-Cyan, R³ Cyano bedeuten und R¹, R² für eine Verbindung jeweils einer Zeile der Tabelle A entspricht

### Tabelle 168

Verbindungen der Formel Ia, Ib, Ic, Id, Ie, If, Ig, Ih und Ii, in denen Lₙ 2,6-Difluor,4-methoxy, R³ Cyano bedeuten und R¹, R² für eine Verbindung jeweils einer Zeile der Tabelle A entspricht

### Tabelle 169

Verbindungen der Formel Ia, Ib, Ic, Id, Ie, If, Ig, Ih und Ii, in denen Lₙ 2-Fluor,3-methyl, R³ Cyano bedeuten und R¹, R² für eine Verbindung jeweils einer Zeile der Tabelle A entspricht

### Tabelle 170

Verbindungen der Formel Ia, Ib, Ic, Id, Ie, If, Ig, Ih und Ii, in denen Lₙ 2,5-Dimethyl, R³ Cyano bedeuten und R¹, R² für eine Verbindung jeweils einer Zeile der Tabelle A entspricht

### Tabelle 171

Verbindungen der Formel Ia, Ib, Ic, Id, Ie, If, Ig, Ih und Ii, in denen Lₙ 2-Methyl,4-cyan, R³ Cyano bedeuten und R¹, R² für eine Verbindung jeweils einer Zeile der Tabelle A entspricht

### Tabelle 172

Verbindungen der Formel Ia, Ib, Ic, Id, Ie, If, Ig, Ih und Ii, in denen Lₙ 2-Methyl, 4-brom, R³ Cyano bedeuten und R¹, R² für eine Verbindung jeweils einer Zeile der Tabelle A entspricht

### Tabelle 173

Verbindungen der Formel Ia, Ib, Ic, Id, Ie, If, Ig, Ih und Ii, in denen Lₙ 2-Methyl,5-fluor, R³ Cyano bedeuten und R¹, R² für eine Verbindung jeweils einer Zeile der Tabelle A entspricht

### Tabelle 174

Verbindungen der Formel Ia, Ib, Ic, Id, Ie, If, Ig, Ih und Ii, in denen Lₙ 2-Methyl,4-methoxy, R³ Cyano bedeuten und R¹, R² für eine Verbindung jeweils einer Zeile der Tabelle A entspricht

### Tabelle 175

Verbindungen der Formel Ia, Ib, Ic, Id, Ie, If, Ig, Ih und Ii, in denen Lₙ 2-Methyl,4-methoxycarbonyl, R³ Cyano bedeuten und R¹, R² für eine Verbindung jeweils einer Zeile der Tabelle A entspricht

### Tabelle 176

Verbindungen der Formel Ia, Ib, Ic, Id, Ie, If, Ig, Ih und Ii, in denen Lₙ 2,5-Dimethyl,4-brom, R³ Cyano bedeuten und R¹, R² für eine Verbindung jeweils einer Zeile der Tabelle A entspricht

### Tabelle 177

Verbindungen der Formel Ia, Ib, Ic, Id, Ie, If, Ig, Ih und Ii, in denen Lₙ 2-Fluor,4-brom, R³ Cyano bedeuten und R¹, R² für eine Verbindung jeweils einer Zeile der Tabelle A entspricht

### Tabelle 178

Verbindungen der Formel Ia, Ib, Ic, Id, Ie, If, Ig, Ih und Ii, in denen Lₙ 2-Fluor,4-methoxy, R³ Cyano bedeuten und R¹, R² für eine Verbindung jeweils einer Zeile der Tabelle A entspricht

### Tabelle 179

Verbindungen der Formel Ia, Ib, Ic, Id, Ie, If, Ig, Ih und Ii, in denen Lₙ 2-Fluor,5-methyl, R³ Cyano bedeuten und R¹, R² für eine Verbindung jeweils einer Zeile der Tabelle A entspricht

### Tabelle 180

Verbindungen der Formel Ia, Ib, Ic, Id, Ie, If, Ig, Ih und Ii, in denen Lₙ Pentafluor, R³ Cyano bedeuten und R¹, R² für eine Verbindung jeweils einer Zeile der Tabelle A entspricht

**Tabelle A**

| **No.** | **R¹** | **R²** |
|---|---|---|
| A-1 | CH₂CH₃ | H |
| A-2 | CH₂CH₃ | CH₃ |
| A-3 | CH₂CH₃ | CH₂CH₃ |
| A-4 | CH₂CH₂CH₃ | H |
| A-5 | CH₂CH₂CH₃ | CH₃ |
| A-6 | CH₂CH₂CH₃ | CH₂CH₃ |
| A-7 | CH₂CH₂CH₃ | CH₂CH₂CH₃ |
| A-8 | CH₂CH₂F | H |
| A-9 | CH₂CH₂F | CH₃ |
| A-10 | CH₂CH₂F | CH₂CH₃ |
| A-11 | CH₂CF₃ | H |
| A-12 | CH₂CF₃ | CH₃ |
| A-13 | CH₂CF₃ | CH₂CH₃ |
| A-14 | CH₂CF₃ | CH₂CH₂CH₃ |
| A-15 | CH₂CCl₃ | H |
| A-16 | CH₂CCl₃ | CH₃ |
| A-17 | CH₂CCl₃ | CH₂CH₃ |
| A-18 | CH₂CCl₃ | CH₂CH₂CH₃ |
| A-19 | CH(CH₃)₂ | H |
| A-20 | CH(CH₃)₂ | CH₃ |
| A-21 | CH(CH₃)₂ | CH₂CH₃ |
| A-22 | CH(CH₃)₂ | CH₂CH₂CH₃ |
| A-23 | CH₂C(CH₃)₃ | H |
| A-24 | CH₂C(CH₃)₃ | CH₃ |
| A-25 | CH₂C(CH₃)₃ | CH₂CH₃ |
| A-26 | CH₂CH(CH₃)₂ | H |
| A-27 | CH₂CH(CH₃)₂ | CH₃ |
| A-28 | CH₂CH(CH₃)₂ | CH₂CH₃ |
| A-29 | (±)CH(CH₂CH₃)CH₃ | H |
| A-30 | (±)CH(CH₂CH₃)CH₃ | CH₃ |
| A-31 | (±)CH(CH₂CH₃)CH₃ | CH₂CH₃ |
| A-32 | (R) CH(CH₂CH₃)CH₃ | H |
| A-33 | (R) CH(CH₂CH₃)CH₃ | CH₃ |
| A-34 | (R) CH(CH₂CH₃)CH₃ | CH₂CH₃ |
| A-35 | (S) CH(CH₂CH₃)CH₃ | H |
| A-36 | (S) CH(CH₂CH₃)CH₃ | CH₃ |
| A-37 | (S) CH(CH₂CH₃)CH₃ | CH₂CH₃ |
| A-38 | (±)CH(CH₃)-CH(CH₃)₂ | H |
| A-39 | (±)CH(CH₃)-CH(CH₃)₂ | CH₃ |
| A-40 | (±) CH(CH₃)-CH(CH₃)₂ | CH₂CH₃ |
| A-41 | (R) CH(CH₃)-CH(CH₃)₂ | H |
| A-42 | (R) CH(CH₃)-CH(CH₃)₂ | CH₃ |
| A-43 | (R) CH(CH₃)-CH(CH₃)₂ | CH₂CH₃ |
| A-44 | (S) CH(CH₃)-CH(CH₃)₂ | H |
| A-45 | (S) CH(CH₃)-CH(CH₃)₂ | CH₃ |
| A-46 | (S) CH(CH₃)-CH(CH₃)₂ | CH₂CH₃ |
| A-47 | (±) CH(CH₃)-C(CH₃)₃ | H |
| A-48 | (±) CH(CH₃)-C(CH₃)₃ | CH₃ |
| A-49 | (±) CH(CH₃)-C(CH₃)₃ | CH₂CH₃ |
| A-50 | (R) CH(CH₃)-C(CH₃)₃ | H |
| A-51 | (R) CH(CH₃)-C(CH₃)₃ | CH₃ |
| A-52 | (R) CH(CH₃)-C(CH₃)₃ | CH₂CH₃ |
| A-53 | (S) CH(CH₃)-C(CH₃)₃ | H |
| A-54 | (S) CH(CH₃)-C(CH₃)₃ | CH₃ |
| A-55 | (S) CH(CH₃)-C(CH₃)₃ | CH₂CH₃ |
| A-56 | (±) CH(CH₃)-CF₃ | H |
| A-57 | (±)CH(CH₃)-CF₃ | CH₃ |
| A-58 | (±) CH(CH₃)-CF₃ | CH₂CH₃ |
| A-59 | (R) CH(CH₃)-CF₃ | H |
| A-60 | (R) CH(CH₃)-CF₃ | CH₃ |
| A-61 | (R) CH(CH₃)-CF₃ | CH₂CH₃ |
| A-62 | (S) CH(CH₃)-CF₃ | H |
| A-63 | (S) CH(CH₃)-CF₃ | CH₃ |
| A-64 | (S) CH(CH₃)-CF₃ | CH₂CH₃ |
| A-65 | (±)CH(CH₃)-CCl₃ | H |
| A-66 | (±) CH(CH₃)-CCl₃ | CH₃ |
| A-67 | (±) CH(CH₃)-CCl₃ | CH₂CH₃ |
| A-68 | (R) CH(CH₃)-CCl₃ | H |
| A-69 | (R) CH(CH₃)-CCl₃ | CH₃ |
| A-70 | (R) CH(CH₃)-CCl₃ | CH₂CH₃ |
| A-71 | (S) CH(CH₃)-CCl₃ | H |
| A-72 | (S) CH(CH₃)-CCl₃ | CH₃ |
| A-73 | (S) CH(CH₃)-CCl₃ | CH₂CH₃ |
| A-74 | CH₂C(CH₃)=CH₂ | H |
| A-75 | CH₂C(CH₃)=CH₂ | CH₃ |
| A-76 | CH₂C(CH₃)=CH₂ | CH₂CH₃ |
| A-77 | Cyclopentyl | H |
| A-78 | Cyclopentyl | CH₃ |
| A-79 | Cyclopentyl | CH₂CH₃ |
| A-80 | Cyclohexyl | H |
| A-81 | Cyclohexyl | CH₃ |
| A-82 | Cyclohexyl | CH₂CH₃ |
| A-83 | -(CH₂)₄- | |
| A-84 | (±) -(CH₂)₂-CH(CH₃)-CH₂- | |
| A-85 | (R) -(CH₂)₂-CH(CH₃)-CH₂- | |
| A-86 | (S) -(CH₂)₂-CH(CH₃)-CH₂- | |
| A-87 | -(CH₂)₂-CH(OCH₃)-CH₂- | |
| A-88 | -(CH₂)₂-CH(CH₂CH₃)-CH₂- | |
| A-89 | -(CH₂)₂-CH[CH(CH₃)₂]-CH₂- | |
| A-90 | (±) -(CH₂)₃-CH(CH₃)- | |
| A-91 | (±) -CH(CH₃)-(CH₂)₂-CH(CH₃)- | |
| A-92 | -CH₂-CH=CH-CH₂- | |
| A-93 | -(CH₂)₅- | |
| A-94 | (±) -(CH₂)₄-CH(CH₃)- | |
| A-95 | -(CH₂)₂-CH(CH₃)-(CH₂)₂- | |
| A-96 | (±) -(CH₂)₃-CH(CH₃)-CH₂- | |
| A-97 | (R) -(CH₂)₃-CH(CH₃)-CH₂- | |
| A-98 | (S) -(CH₂)₃-CH(CH₃)-CH₂- | |
| A-99 | -(CH₂)₂-C(O[CH₂]₂O)-(CH₂)₂- | |
| A-100 | 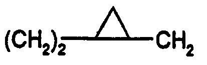 | |
| A-101 | -(CH₂)₂-C(O[CH₂]₃O)-(CH₂)₂- | |
| A-102 | -(CH₂)₂-CH=CH-CH₂- | |

Die Verbindungen I eignen sich als Fungizide. Sie zeichnen sich aus durch eine hervorragende Wirksamkeit gegen ein breites Spektrum von pflanzenpathogenen Pilzen, insbesondere aus der Klasse der Ascomyceten, Deuteromyceten, Oomyceten und Basidiomyceten. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
*Alternaria*-Arten an Gemüse und Obst,
*Bipolaris-* und *Drechslera*-Arten an Getreide, Reis und Rasen,
*Blumeria graminis* (echter Mehltau) an Getreide,
*Botrytis cinerea* (Grauschimmel) an Erdbeeren, Gemüse, Zierpflanzen und Reben,
*Erysiphe cichoracearum* und *Sphaerotheca fuliginea* an Kürbisgewächsen,
*Fusarium-* und *Verticillium*-Arten an verschiedenen Pflanzen,
Mycosphaerella-Arten an Getreide, Bananen und Erdnüssen,
*Phytophthora infestans* an Kartoffeln und Tomaten,
*Plasmopara viticola* an Reben,
*Podosphaera leucotricha* an Äpfeln,
*Pseudocercosporella* herpotrichoides an Weizen und Gerste,
*Pseudoperonospora-Arten* an Hopfen und Gurken,
*Puccinia*-Arten an Getreide,
*Pyricularia oryzae* an Reis,
*Rhizoctonia*-Arten an Baumwolle, Reis und Rasen,
*Septoria tritici* und *Stagonospora nodorum* an Weizen,
*Uncinula necator* an Reben,
*Ustilago*-Arten an Getreide und Zuckerrohr, sowie
*Venturia*-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:
Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
   - Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind: 1. Produkte zur Verdünnung in Wasser

### A) Wasserlösliche Konzentrate (SL)

10 Gew.-Teile einer erfindungsgemäßen Verbindung werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff.

### B) Dispergierbare Konzentrate (DC)

20 Gew.-Teile einer erfindungsgemäßen Verbindung werden in Cyclohexanon unter Zusatz eines Dispergiermittels z.B. Polyvinylpyrrolidon gelöst. Bei Verdünnung in Wasser ergibt sich eine Dispersion.

### C) Emulgierbare Konzentrate (EC)

15 Gew.-Teile einer erfindungsgemäßen Verbindung werden in Xylol unter Zusatz von Ca-Dodecylbenzolsulfonat und Ricinusölethoxylat (jeweils 5 %) gelöst. Bei der Verdünnung in Wasser ergibt sich eine Emulsion.

### D) Emulsionen (EW, EO)

40 Gew.-Teile einer erfindungsgemäßen Verbindung werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.

### E) Suspensionen (SC, OD)

20 Gew.-Teile einer erfindungsgemäßen Verbindung werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.

### F) Wasserdispergierbare und wasserlösliche Granulate (WG, SG)

50 Gew.-Teile einer erfindungsgemäßen Verbindung werden unter Zusatz von Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.

### G) Wasserdispergierbare und wasserlösliche Pulver (WP, SP)

75 Gew.-Teile einer erfindungsgemäßen Verbindung werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs.

### 2. Produkte für die Direktapplikation

### H) Stäube (DP)

5 Gew.Teile einer erfindungsgemäßen Verbindung werden fein gemahlen und mit 95 % feinteiligem Kaolin innig vermischt. Man erhält dadurch ein Stäubemittel.

### I) Granulate (GR, FG, GG, MG)

0.5 Gew-Teile einer erfindungsgemäßen Verbindung werden fein gemahlen und mit 95.5 % Trägerstoffe verbunden. Gängige Verfahren sind dabei die Extrusion, die Sprühtrocknung oder die Wirbelschicht. Man erhält dadurch ein Granulat für die Direktapplikation.

### J) ULV- Lösungen (UL)

10 Gew.-Teile einer erfindungsgemäßen Verbindung werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Acylalanine wie Benalaxyl, Metalaxyl, Ofurace, Oxadixyl,
- Aminderivate wie Aldimorph, Dodine, Dodemorph, Fenpropimorph, Fenpropidin, Guazatine, Iminoctadine, Spiroxamin, Tridemorph
- Anilinopyrimidine wie Pyrimethanil, Mepanipyrim oder Cyrodinyl,
- Antibiotika wie Cycloheximid, Griseofulvin, Kasugamycin, Natamycin, Polyoxin oder Streptomycin,
- Azole wie Bitertanol, Bromoconazol, Cyproconazol, Difenoconazole, Dinitroconazol, Epoxiconazol, Fenbuconazol, Fluquiconazol, Flusilazol, Flutriafol, Hexaconazol, Imazalil, Metconazol, Myclobutanil, Penconazol, Propiconazol, Prochloraz, Prothioconazol, Tebuconazol, Triadimefon, Triadimenol, Triflumizol, Triticonazol,
- Dicarboximide wie lprodion, Myclozolin, Procymidon, Vinclozolin,
- Dithiocarbamate wie Ferbam, Nabam, Maneb, Mancozeb, Metam, Metiram, Propineb, Polycarbamat, Thiram, Ziram, Zineb,
- Heterocylische Verbindungen wie Anilazin, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, lsoprothiolan, Mepronil, Nuarimol, Probenazol, Proquinazid, Pyrifenox, Pyroquilon, Quinoxyfen, Silthiofam, Thiabendazol, Thifluzamid, Thiophanat-methyl, Tiadinil, Tricyclazol, Triforine,
- Kupferfungizide wie Bordeaux Brühe, Kupferacetat, Kupferoxychlorid, basisches Kupfersulfat,
- Nitrophenylderivate, wie Binapacryl, Dinocap, Dinobuton, Nitrophthal-isopropyl
- Phenylpyrrole wie Fenpiclonil oder Fludioxonil,
- Schwefel
- Sonstige Fungizide wie Acibenzolar-S-methyl, Benthiavalicarb, Carpropamid, Chlorothalonil, Cyflufenamid, Cymoxanil, Dazomet, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminium, Iprovalicarb, Hexachlorbenzol, Metrafenon, Pencycuron, Propamocarb, Phthalid, Tolclofos-methyl, Quintozene, Zoxamid
- Strobilurine wie Azoxystrobin, Dimoxystrobin, Fluoxastrobin, Kresoxim-methyl, Metominostrobin, Orysastrobin, Picoxystrobin, Pyraclostrobin oder Trifloxystrobin,
- Sulfensäurederivate wie Captafol, Captan, Dichlofluanid, Folpet, Tolylfluanid
- Zimtsäureamide und Analoge wie Dimethomorph, Flumetover oder Flumorph.

### Synthesebeispiele

### Beispiel 1:

### Synthese von 1-[4-Chloro-6-(2,2,2-trifluor-1-methyl-ethylamino)-5-(2,4,6-trifluorophenyl)-pyrimidin-2-yl]-pyrrolidin-2-on [I-2]

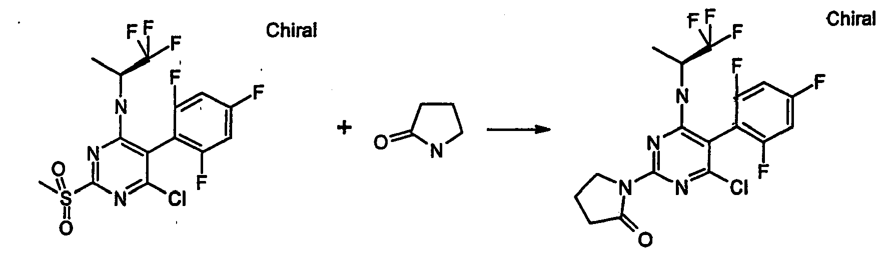

Die Synthese wurde analog MCALONAN, H.; MONTGOMERY, D.; STEVENSON, P. J.; Tetrahedron Lett. 1996, 37 (39), 7151-7154 durchgeführt.
206 mg (2.4207 mmol) 2-Pyrrolidinon wurden in 5 ml THF p.A auf -78°C abgekühlt, dazu tropfte man bei -78°C 2.7665 mmol einer 2M LDA-Lösung in THF/n-Hexan zu und ließ 15 min. nachrühren. Danach tropfte man 1000 mg (2.3054 mmol) des oben aufgeführten Sulfons gelöst in 5 ml THF p.A. hinzu, entfernte das Trockeneiskältebad und ließ über Nacht bei Raumtemperatur rühren.
Man fügte destilliertes Wasser hinzu, extrahierte mit Methylter.butylether, trocknete die organische Phase über MgSO4 und rotierte ein. Es wurde ein leicht beige gefärbter Feststoff in 65% Ausbeute erhalten.
MSD :Produktmasse M=439(+H);
Fp.: 142-145°C

### Beispiel 2:

### Synthese von 1-[4-Chloro-6-(2,2,2-trifluor-1-methyl-ethylamino)-5-(2-chloro-6-fluorophenyl)-pyrimidin-2-yl]-pyridazolidin-3-on [I-7]

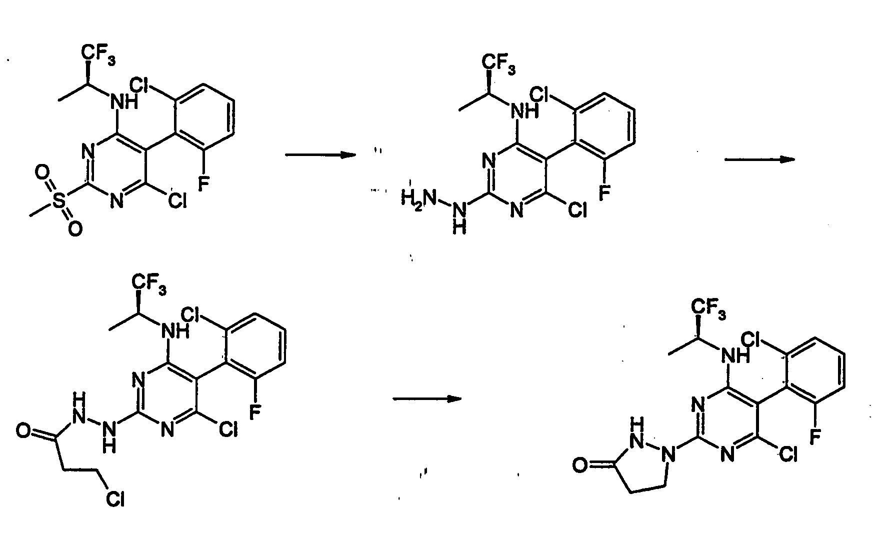

### 2.1) [4-Chloro-2-hydrazino-5-(2-chloro-6-fluorophenyl)-pyrimidin-6-yl]-(2,2,2-trifluor-1-methyl-ethyl)-amin

18,1 g (0,042 mol) des Sulfons [4-Chloro-2-methylsufenyl-5-(2-chloro-6-fluorophenyl)-pyrimidin-6-yl]-(2,2,2-trifluor-1-methyl-ethyl)-amin wurden in 100 ml Ethanol vorgelegt und zu dieser Mischung wurden 4,7 g (0,093 mol) Hydrazin-hydrat zugetropft. Es wurde über Nacht bei Raumtemperatur nachgerührt. Die Mischung wurde eingeengt und der Rückstand in Essigester gelöst, mit Wasser gewaschen, Ober Magnesiumsulfat getrocknet und eingeengt. Der Rückstand wurde mit Diisopropylether verrieben, die Kristalle abgesaugt, mit Diisopropylether und n-Pentan gewaschen und im Vakuum getrocknet. Man erhielt 10,8 g (67% Ausbeute) eines Feststoffes mit einem Schmelzpunkt von 120-122 °C.

2.2) 3-Chloro-propionsäure-N'-[4-chloro-6-(2,2,2-trifluor-1-methyl-ethylamino)-5-(2-chloro-6-fluorophenyl)-pyrimidin-2-yl]-hydrazid

0,60 g (1,6 mmol) der in 2.1 synthetisierten Verbindung wurden in 10 ml absolutem Methylenchlorid gelöst und mit 0,20 g (2,4 mmol) Pyridin versetzt. Anschließend wurde bei 0-10°C 0,25 g (1,9 mmol) 3-Chlorpropionsäurechlorid in 1 ml absolutem Methylenchlorid zugetropft. Es wurde über Nacht bei Raumtemperatur nachgerührt, die Mischung mit Methylenchlorid verdünnt und nacheinander mit gesättigter Natriumhydrogencarbonatlösung, fünfprozentiger Essigsäure und mit Wasser gewaschen, über Magnesiumsulfat getrocknet und eingeengt. Der Rückstand wurde durch Flashchromatographie an Kieselgel mit Methylter.butylether/Hexan gereinigt. Man erhielt 0,5 g (66% Ausbeute) eines farblosen Feststoffes mit einem Schmelzpunkt von 152-156 °C.

### 2.3) Synthese von 1-[4-Chloro-6-(2,2,2-trifluor-1-methyl-ethylamino)-5-(2-chloro-6-fluorophenyl)-pyrimidin-2-yl]-pyridazolidin-3-on

Unter Schutzgas wurden 130 mg (1,3 mmol) Diisopropylamin und 5 ml absolutes THF vorgelegt und bei -78°C 0,8 ml (1,3 mmol) einer 1,6 molaren Butyllithiumlösung in Hexan zugetropft. Es wurde 30 Minuten bei-78 °C nachgerührt und zu einer Lösung von 0,3 g (0,63 mmol) des Amids 2.2 in 5 ml absoluten THF zugetropft. Die Mischung wurde innerhalb von 3-4 Stunden auf Raumtemperatur erwärmt und über Nacht bei Raumtemperatur nachgerührt. Es wurde mit Wasser versetzt, mit Methylter.butylether extrahiert, mit Wasser gewaschen, über Magnesiumsulfat getrocknet und eingeengt. Die Reinigung über präparative HPLC lieferte 50 mg eines gelben Feststoffes (18% Ausbeute) mit einem Schmelzpunkt von 214-217 °C.

Die in den vorstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle I mit physikalischen Daten aufgeführt.

**Tabelle I**

| 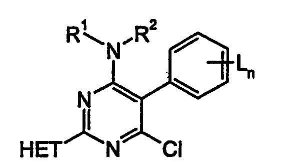 | | | | | |
|---|---|---|---|---|---|
| **Nr.** | **HET** | **R¹** | **R²** | **Lₙ** | **Fp. [°C]** |
| I-1 | 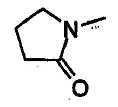 | -CH₂CH=CH₂ | -(CH₂)₂CH₃ | 2-Chlor-4-fluor | 124-127 |
| I-2 | 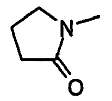 | (S) -CH(CH₃)CF₃ | H | 2,4,6-Trifluor | 141-145 |
| I-4 | 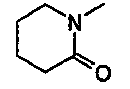 | (S) -CH(CH₃)CF₃ | H | 2,4,6-Trifluor | 122-127 |
| I-5 | 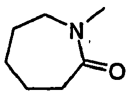 | (S) -CH(CH₃)CF₃ | H | 2,4,6-Trifluor | 168-170 |
| I-6 | 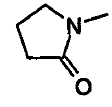 | -CH₂CF₃ | H | 2,4,6-Trifluor | 192-193 |
| I-7 | 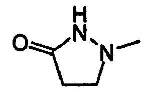 | (S) -CH(CH₃)CF₃ | H | 2-Chlor-6-fluor | 214-217 |
| I-8 | 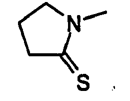 | (S) -CH(CH₃)CF₃ | H | 2,4,6-Trifluor | 86-94 |
| I-9 | 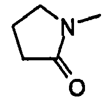 | (R) -CH(CH₃)CF₃ | H | 2,4,6-Trifluor | 142-145 |
| I-10 | 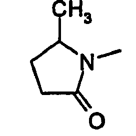 | (S) -CH(CH₃)CF₃ | H | 2,4,6-Trifluor | 65-81 |
| I-11 | 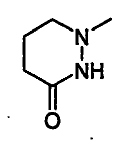 | (S) -CH(CH₃)CF₃ | H | 2-Chlor-6-fluor | 1H-NMR: δ (ppm, COCl₃) = 1,3 (s, 3H); 2,15 (m, 2H); 2,5 (m, 2H); 3,75 (m, 2H); 4,3 (m, 1H); 4,92 (m, 1H); 7,18 (m, 1H); 7,4 (m, 2H) |
| I-12 | 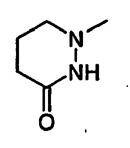 | -CH(CH₃)C₂H₅ | H | 2-Chlor-6-fluor | H-NMR: δ (ppm, CDCl₃) = 0,83 (m, 3H); 1,12 (m, 3H); 1,45 (m, 2H); 2,15 (m, 2H); 2,48 (m, 2H); 3,75 (m, 2H); 4,2 (s, 1 H); 4,33 (m, 1H); 7,15(m, 1H); 7,4 (m, 2H) |
| I-13 | 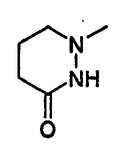 | -(CH₂)₂-CH(CH₃)-(CH₂)₂- | | 2-Chlor-6-fluor | H-NMR: ö (ppm, CDCl₃) = 0,88 (d, 3H); 1,0 (q, 2H); 1,45 (s, 1 H); 1,49 (s, 2H); 2,13 (m, 2H); 2,48 (t, 2H); 2,68 (t, 2H); 3,75 (t, 2H); 3,85 (t, 2H); 7,08 (t, 1H); 7,3 (m, 2H) |
| I-14 | 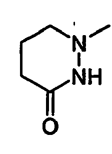 | -CH(CH₃)C₂H₃ | H | 2,4,6-Trifluor | 1H-NMR: δ (ppm, CDCl₃) = 0,85 (t, 3H); 1,1 (d, 3); 1,45 (m, 2H); 2,15 (m, 2H); 2,47 (t, 2H); 3,75 (t, 2H); 4,0 (m, 1 H); 4,2 (m, 1 H); 6,8 (t, 2H) |
| I-15 | 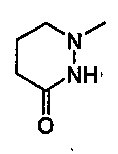 | -(CH₂)₂-CH(CH₃)-(CH₂)₂- | | 2,4,6-Trifluor | 1 H-NMR: δ (ppm, CDCl₃) = 0,88 (m, 3H); 1,0 (q, 2H); 1,5 (s, 1H); 1,52 (s, 2H); 2,15 (m, 2H);; 2,48 (t, 2H); 2,72 (m, 2H); 2,72 (t, 2H); 3,85 (d, 2H); 6,75 (m, 2H) |
| I-16 | 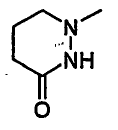 | (S)-CH(CH₃)CF₃ | H | 2,4,6-Trifluor | 1 H-NMR: δ (ppm, CDCl₃) = 1,3 (d, 3H); 2,16 (m, 2H); 2,5 (m, 2H); 3,72 (m, 2H); 4,35 (m, 1 H); 4,9 (m, 1 H); 6,85 (m, 2H) |
| I-17 | 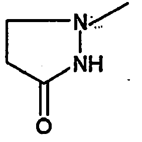 | (S)-CH(CH₃)CF₃ | H | 2,4,6-Trifluor | 1 H-NMR: δ (ppm, Aceton) = 1,5 (d, 3H); 2,75 (m, 2H); 4,2 (t, 2H); 5,6 (m, 1 H); 6,62 (d, 1 H); 7,08 (m, 2H) |
| I-18 | 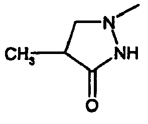 | (S)-CH(CH₃)CF₃ | H | 2,4,6-Trifluor | 200 |
| I-19 | 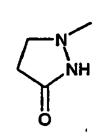 | (S)-CH(CH₃)C₂H₅ | H | 2-Chlor-6-fluor | 1H-NMR: δ (ppm, CDCl₃) = 0,85 (m, 3H); 1,1 (m, 3H); 1,45 (m, 2H); 2,8 (t, 2H); 4,12 (s, 2H); 4,25 (t, 2H);7,15 (m, 1 H); 7,38 (m, 2H |
| I-20 | 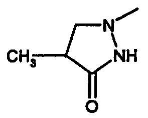 | (S)-CH(CH₃)CF₃ | H | 2-Chlor6-fluor | 195 - 198 |
| I-21 | 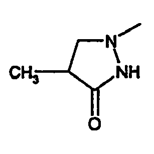 | -CH(CH₃)C₂H₅ | H | 2-Chlor- 6-fluor | 1 H-NMR: δ (ppm, CDCl₃) = 0,86 (m, 3H); 1,11 (t, 3H); 1,36 (d, 3H); 1,45 (m, 2H); 2,97 (m, 1 H); 3,65 (t, 1 H); 4,1 (s, 2H); 4,55 (t, 1 H); 7,15 (t, 1 H); 7,39 (m, 2H) |
| I-22 | 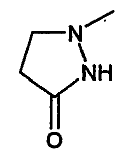 | -(CH₂)₂-CH(CH₃)-(CH₂)₂- | | 2-Chlor-6-fluor | 142-145 |
| I-23 | 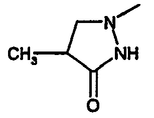 | -(CH₂)₂-CH(CH₃)-(CH₂)₂- | | 2-Chlor-6-fluor | 180 - 183 |
| I-24 | 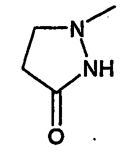 | -CH(CH₃)C₂H₅ | H | 2,4,6-Trifluor | 205 - 208 |
| I-25 | 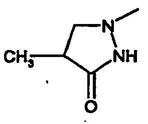 | -CH(CH₃)C₂H₅ | H | 2,4,6-Trifluor | 193 - 196 |
| I-26 | 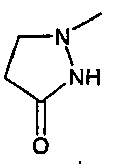 | -(CH₂)₂-CH(CH₃)-(CH₂)₂- | | 2,4,6-Trifluor | 1 H-NMR: δ (ppm, CDCl₃) = 0,9 (d, 3H); 1,0 (q, 2H); 1,5 (s, 1H); 1,52 (s, 2H); 2,7 (t, 2H); 2,8 (t, 2H); 3,85 (d, 2H); 4,2 (t, 2H); 6,75 (m, 2H) |
| I-27 | 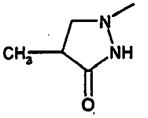 | -(CH₂)₂-CH(CH₃)-(CH₂)₂- | | 2,4,6-Trifluor | 185 - 188 |

### Beispiele für die Wirkung gegen Schadpilze

Die fungizide Wirkung der Verbindungen der Formel I ließ sich durch die folgenden Versuche zeigen:

Die Wirkstoffe wurden getrennt als Stammlösung formuliert mit 0,25 Gew.-% Wirkstoff in Aceton oder DMSO. Dieser Lösung wurde 1 Gew.-% Emulgator Uniperol® EL (Netzmittel mit Emulgier- und Dispergierwirkung auf Basis ethoxylierter Alkylphenole) zugesetzt. Die Stammlösungen der Wirkstoffe wurden entsprechend der angegebenen Konzentration mit Wasser verdünnt

### Anwendungsbeispiele

### 1. Wirksamkeit gegen den Grauschimmel an Paprikablättem verursacht durch Botrytis cinerea bei protektiver Behandlung

Paprikasämlinge der Sorte "Neusiedler Ideal Elite" wurden, nachdem sich 4-5 Blätter gut entwickelt hatten, mit einer wässrigen Suspension in einer Wirkstoffkonzentration von 250 ppm bis zur Tropfnässe besprüht. Am nächsten Tag wurden die behandelten Pflanzen mit einer Sporensuspension von *Botrytis cinerea*, die 1.7 x 10⁶ Sporen/ml in einer 2 %igen wässrigen Biomalzlösung enthielt, inokuliert. Anschließend wurden die Versuchspflanzen in eine Klimakammer mit 22 bis 24°C und hoher Luftfeuchtigkeit gestellt. Nach 5 Tagen konnte das Ausmaß des Pilzbefalls auf den Blättern visuell in % ermittelt werden.

Bei diesem Versuch zeigten die mit Verbindungen I-2, I-4, I-6, I-7, I-9, I-11, I-17, I-18, I-19, I-20, I-21, I-22, I-24 und I-25 behandelten Pflanzen einen Befall von kleiner 5% während die unbehandelten Pflanzen zu 90% befallen waren.

### 2. Wirksamkeit gegen die Dürrfleckenkrankheit der Tomate verursacht durch Altemaria solani

Blätter von Topfpflanzen der Sorte "Goldene Prinzessin" wurden mit einer wässriger Suspension in der unten angegebenen Wirkstoffkonzentration bis zur Tropfnässe besprüht Am folgenden Tag wurden die Blätter mit einer wässrigen Sporenaufschwemmung von *Alternaria solani* in 2 % Biomalzlösung mit einer Dichte von 0.17 x 10⁶ Sporen/ml infiziert. Anschließend wurden die Pflanzen in einer wasserdampfgesättigten Kammer bei Temperaturen zwischen 20 und 22°C aufgestellt. Nach 5 Tagen hatte sich die Krautfäule auf den unbehandelten, jedoch infizierten Kontrollpflanzen so stark entwickelt, dass der Befall visuell in % ermittelt werden konnte.

Bei diesem Versuch zeigten die mit Verbindungen I-2, I-4, I-6 und I-7 behandelten Pflanzen einen Befall von kleiner 10% während die unbehandelten Pflanzen zu 90% befallen waren.

### 3. Wirksamkeit gegen die Netzfleckenkrankheit der Gerste verursacht durch Pyrenophora teres bei 1 Tag protektiver Anwendung

Blätter von in Töpfen gewachsenen Gerstenkeimlingen wurden mit wässriger Suspension in der unten angegebenen Wirkstoffkonzentration bis zur Tropfnässe besprüht. 24 Stunden nach dem Antrocknen des Spritzbelages wurden die Versuchspflanzen mit einer wässrigen Sporensuspension von *Pyrenophora [syn. Drechslera] teres,* dem Erreger der Netzfleckenkrankheit inokuliert. Anschließend wurden die Versuchspflanzen im Gewächshaus bei Temperaturen zwischen 20 und 24°C und 95 bis 100 % relativer Luftfeuchtigkeit aufgestellt. Nach 6 Tagen wurde das Ausmaß der Krankheitsentwicklung visuell in % Befall der gesamten Blattfläche ermittelt.

Bei diesem Versuch zeigten die mit Verbindungen I-11, I-17, I-18, I-19, I-20, I-21, I-22, I-24 und I-25 behandelten Pflanzen einen Befall von kleiner 20 %, während die unbehandelten Pflanzen zu 90 % befallen waren.

## Patentansprüche

1. 2-Substituierte Pyrimidine der Formel I 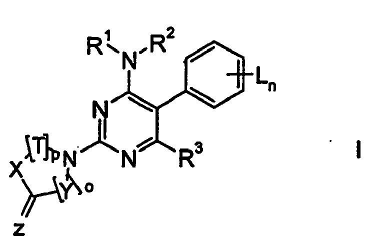 in der die Indices und die Substituenten die folgende Bedeutung haben:
n eine ganze Zahl von 1 bis 5;
L Halogen, Cyano, Cyanato (OCN), C₁-C₈-Alkyl, C₂-C₈-Alkenyl, C₂-C₈-Alkinyl, C₁-C₆-Alkoxy, C₂-C₈-Alkenyloxy, C₂-C₈-Alkinyloxy, C₃-C₆-Cycloalkyl, C₄-C₆-Cycloalkenyl, C₃-C₆-Cycloalkyloxy, C₄-C₆-Cycloalkenyloxy, Nitro, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A,
m 0, 1 oder 2;
A, A', A" unabhängig voneinander Wasserstoff, C₁-C₆-Alkyl, C₂-C₆-Alkenyl, C₂-C₆-Alkinyl, C₃-C₈-Cycloalkyl, C₃-C₈-Cycloalkenyl, Phenyl, wobei die organischen Reste partiell oder vollständig halogeniert sein können oder durch Nitro, Cyanato, Cyano oder C₁-C₄-Alkoxy substituiert sein können; oder A und A' zusammen mit den Atomen an die sie gebunden sind für einen fünf- bis sechsgliedrigen gesättigten, partiell ungesättigten oder aromatischen Heterocyclus, enthaltend ein bis vier Heteroatome aus der Gruppe O, N oder S, stehen;
wobei die aliphatischen Gruppen der Restedefinitionen von L ihrerseits partiell oder vollständig halogeniert sein oder eine bis vier Gruppen R^{u} tragen können:
R^{u} Cyano, C₁-C₆-Alkoxy, C₃-C₆-Cycloalkyl, C₂-C₈-Alkenyloxy, C₂-C₈-Alkinyloxy, C₄-C₆-Cycloalkenyl, C₃-C₆-Cycloalkyloxy, C₄-C₆-Cycloalkenyloxy -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A;
R¹, R² unabhängig voneinander C₁-C₆-Alkyl, C₂-C₆-Alkenyl, C₂-C₆-Alkinyl, C₃-C₆-Cycloalkyl, C₃-C₆-Halogencycloalkyl, wobei die aliphatischen Gruppen der Restedefinitionen von R¹ und R² ihrerseits partiell oder vollständig halogeniert sein oder eine bis vier Gruppen R^{v} tragen können:
R^{v} Cyano, C₃-C₆-Cycloalkyl, C₄-C₆-Cycloalkenyl, Hydroxy, C₁-C₆-Alkoxy, C₂-C₈-Alkenyloxy, C₂-C₈-Alkinyloxy, C₃-C₆-Cycloalkyloxy, C₄-C₆-Cycloalkenyloxy, C₁-C₆-Alkylthio, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A oder Phenyl, wobei der Phenylteil ein bis drei Reste ausgewählt aus der Gruppe: Halogen, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy, Cyano, Nitro, -C(=O)-A, -C(=O)-O-A, -C(=O)-N(A')A, C(A')(=N-OA), N(A')A tragen kann;
R² kann zusätzlich Wasserstoff bedeuten;
R¹ und R² können auch zusammen mit dem Stickstoffatom, an das sie gebunden sind, einen gesättigten oder ungesättigten fünf- oder sechsgliedrigen Ring bilden, der durch eine Ether -(-O-), Carbonyl -(C=O)-, Thio -(-S-), Sulfoxyl -(-S[=O]-) oder Sulfenyl -(-SO₂-) oder eine weitere Amino -(-N(R^{a})- Gruppe, wobei R^{a} Wasserstoff oder C₁-C₆-Alkyl bedeutet, unterbrochen sein und/oder einen oder mehrere Substituenten aus der Gruppe Halogen, C₁-C₆-Alkyl, C₁-C₆-Halogenalkyl und Oxy-C₁-C₃-alkylenoxy enthalten kann;
R³ Halogen, Cyano, C₁-C₄-Alkyl, C₂-C₄-Alkenyl, C₂-C₄-Alkinyl, C₃-C₆-Cycloalkyl, C₁-C₄-Alkoxy, C₃-C₄-Alkenyloxy, C₃-C₄-Alkinyloxy, C₁-C₆-Alkylthio, Di-(C₁-C₆-alkyl)amino oder C₁-C₆-Alkylamino, wobei die Alkyl, Alkenyl und Alkinylreste von R³ durch Halogen, Cyano, Nitro, C₁-C₂-Alkoxy oder C₁-C₄-Alkoxycarbonyl substituiert sein können;
X eine Gruppe -CH-R^{a}-, -N-R^{b}-, -O- oder-S-;
R^{a} Wasserstoff, Halogen, C₁-C₆-Alkyl, C₁-C₆-Alkoxy, Cyano oder C₁-C₆-Alkoxycarbonyl sein kann;
R^{b} Wasserstoff, C₁-C₆-Alkyl oder C₃-C₆-Cycloalkyl;
T eine Gruppe -CH-R^{a}- ;
p eine Zahl von 1 bis 4;
Y eine Gruppe -CH-R^{a}- oder -N-R^{b}-'
o 0 oder 1;
Z O, S oder eine Gruppe N(R^{c})
R^{c} Wasserstoff, C₁-C₆-Alkyl oder C₁-C₆-Alkoxy.

2. 2-Substituierte Pyrimidine der Formel I nach Anspruch 1, wobei
n eine ganze Zahl von 1 bis 3 ist, wobei mindestens ein Substituent L in ortho-Stellung am Phenylring sitzt;
L Halogen, Cyano, Methyl, Methoxy, -C(=O)-O-A, -C(=O)-N(A')A, C(A')(=N-OA), N(A')A, N(A')-C(=O)-A,
A,A' unabhängig voneinander Wasserstoff, C₁-C₆-Alkyl, C₂-C₆-Alkenyl, C₂-C₆-Alkinyl, Phenyl, wobei die organischen Reste partiell oder vollständig halogeniert sein können oder durch C₁-C₄-Alkoxy substituiert sein können, oder A und A' zusammen mit den Atomen an die sie gebunden sind für einen fünf- bis sechsgliedrigen gesättigten Heterocyclus, enthaltend ein oder zwei Heteroatome aus der Gruppe O, N oder S stehen;
wobei die aliphatischen Gruppen der Restedefinitionen von L ihrerseits partiell oder vollständig halogeniert sein können;
R¹,R² unabhängig voneinander C₁-C₆-Alkyl, C₂-C₆-Alkenyl, C₂-C₆-Alkinyl, C₁-C₆-Halogenalkyl, C₂-C₆-Halogenalkenyl oder C₂-C₆-Halogenalkinyl;
R² kann zusätzlich Wasserstoff bedeuten;
R¹ und R² können auch zusammen mit dem Stickstoffatom, an das sie gebunden sind, einen gesättigten oder ungesättigten fünf- oder sechsgliedrigen Ring bilden, der durch eine Ether -(-O-) oder eine weitere Amino-(-N(R^{a})- Gruppe, wobei R^{a} Wasserstoff oder C₁-C₆-Alkyl bedeutet, unterbrochen sein und/oder einen oder mehrere Substituenten aus der Gruppe Halogen, C₁-C₆-Alkyl, C₁-C₆-Halogenalkyl und Oxy-C₁-C₃-alkylenoxy enthalten kann;
R³ Halogen, Cyano, C₁-C₄-Alkyl, C₁-C₄-Alkoxy oder C₁-C₄-Haloalkyl;
X eine Gruppe -CH-R^{a}- ;
R^{a} Wasserstoff oder C₁-C₃-Alkyl,
p eine Zahl von 2 bis 4 und
o 0 bedeutet.

3. 2-Substituierte Pyrimidine nach Anspruch 1, wobei R³ Chlor, Cyano, Methyl oder Methoxy bedeutet.

4. 2-Substituierte Pyrimidine nach Anspruch 1, wobei
X eine Gruppe -CH-R^{a}-;
R^{a} Wasserstoff, Halogen, Cyan oder C₁-C₃-Alkyl;
T eine Gruppe -CH-R^{a}- ;
p eine Zahl von 1 bis 3;
Y eine Gruppe-N-R^{b}
R^{b} Wasserstoff oder C₁-C₃-Alkyl und
o 1 bedeutet.

5. 2-Substituierte Pyrimidine nach einem der Ansprüche 1 bis 4, in der die durch Lₙ 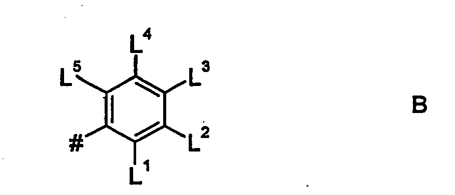 steht, worin # die Verknüpfungsstelle mit dem Pyrimidin-Gerüst ist und
L¹ Fluor, Chlor, CH₃ oder CF₃;
L²,L⁴ unabhängig voneinander Wasserstoff, CH₃ oder Fluor;
L³ Wasserstoff, Fluor, Chlor, Cyano, Nitro, CH₃, SCH₃, OCH₃, SO₂CH₃, NH-C(=O)CH₃, N(CH₃)-C(=O)CH₃ oder COOCH₃ und
L⁵ Wasserstoff, Fluor, Chlor oder CH₃ bedeuten.

6. Verfahren zur Herstellung der Verbindungen IA gemäß Anspruch 1, durch Umsetzung des Sulfons II mit dem Nukleophil III, wobei der Index p 2 bis 5 bedeutet, unter basischen Bedingungen zum Lactam IA.

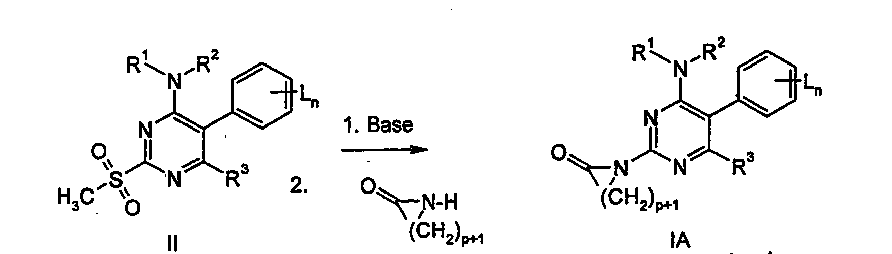

7. Verfahren zur Herstellung der Verbindungen IB gemäß Anspruch 1, durch Umsetzung des Hydrazins IV mit einem Chloralkylcarbonsäurechlorid, wobei der Index p 2 oder 3 bedeutet, zur Verbindung V und anschließendem Ringschluß zum Pyrazolon IB.

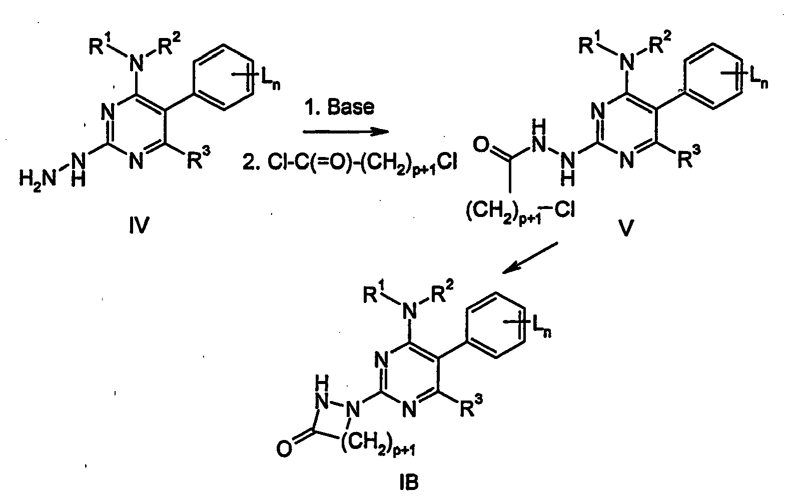

8. Pestizides Mittel, enthaltend einen festen oder flüssigen Trägerstoff und eine Verbindung der Formel I gemäß Anspruch 1.

9. Verfahren zur Bekämpfung von pflanzenpathogenen Schadpilzen, **dadurch gekennzeichnet, dass** man die Pilze oder die vor Pilzbefall zu schützenden Materialien, Pflanzen, den Boden oder Saatgüter mit einer wirksamen Menge einer Verbindung der Formel I gemäß Anspruch 1 behandelt.

## Claims

1. A 2-substituted pyrimidine of the formula I 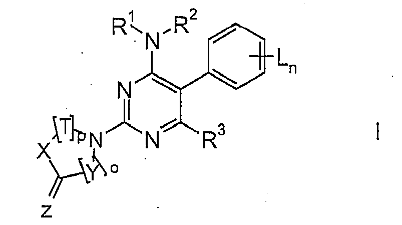 in which the indices and the substituents are as defined below:
n is an integer from 1 to 5;
L is halogen, cyano, cyanato (OCN), C₁-C₈-alkyl, C₂-C₈-alkenyl, C₂-C₈-alkynyl, C₁-C₆-alkoxy, C₂-C₈-alkenyloxy, C₂-C₈-alkynyloxy, C₃-C₆-cycloalkyl, C₄-C₆-cycloalkenyl, C₃-C₆-cycloalkyloxy, C₄-C₆-cycloalkenyloxy, nitro, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A or S(=O)ₘ-N(A')A,
m is 0, 1 or 2;
A, A', A" independently of one another are hydrogen, C₁-C₆-alkyl, C₂-C₆-alkenyl, C₂-C₆-alkynyl, C₃-C₈-cycloalkyl, C₃-C₈-cycloalkenyl, phenyl, where the organic radicals may be partially or fully halogenated or may be substituted by nitro, cyanato, cyano or C₁-C₄-alkoxy; or A and A' together with the atoms to which they are attached are a five- or six-membered saturated, partially unsaturated or aromatic heterocycle which comprises one to four heteroatoms from the group consisting of O, N and S;
where the aliphatic groups of the radical definitions of L for their part may be partially or fully halogenated or may carry one to four groups R^{u}:
R^{u} is cyano, C₁-C₆-alkoxy, C₃-C₆-cycloalkyl, C₂-C₈-alkenyloxy, C₂-C₈-alkynyloxy, C₄-C₆-cycloalkenyl, C₃-C₆-cycloalkyloxy, C₄-C₆-cycloalkenyloxy
-C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A or S(=O)ₘ-N(A')A;
R¹, R² independently of one another are C₁-C₆-alkyl, C₂-C₆-alkenyl, C₂-C₆-alkynyl, C₃-C₆-cycloalkyl, C₃-C₆-halocycloalkyl, where the aliphatic groups of the radical definitions of R¹ and R² for their part may be partially or fully halogenated or may carry one to four groups R^{v}:
R^{v} is cyano, C₃-C₆-cycloalkyl, C₄-C₆-cycloalkenyl, hydroxyl, C₁-C₆-alkoxy, C₂-C₈-alkenyloxy, C₂-C₈-alkynyloxy, C₃-C₆-cycloalkyloxy, C₄-C₆-cycloalkenyloxy, C₁-C₆-alkylthio, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A or S(=O)ₘ-N(A')A or phenyl, where the phenyl moiety may carry one to three radicals selected from the group consisting of: halogen, C₁-C₆-alkyl, C₂-C₆-alkenyl, C₂-C₆-alkynyl, C₃-C₆-cycloalkyl, C₁-C₆-haloalkyl, C₁-C₆-alkoxy, cyano, nitro, -C(=O)-A, -C(=O)-O-A, -C(=O)-N(A')A, C(A')(=N-OA), N(A')A;
R² may additionally be hydrogen;
R¹ and R² may also, together with the nitrogen atom to which they are attached, form a saturated or unsaturated five- or six-membered ring which may be interrupted by an ether -(-O-), carbonyl -(C=O)-, thio -(-S-), sulfoxyl -(-S[=O]-) or sulfenyl -(-SO₂-) group or by a further amino -(-N(R^{a})- group, where R^{a} is hydrogen or C₁-C₆-alkyl, and/or may comprise one or more substituents from the group consisting of halogen, C₁-C₆-alkyl, C₁-C₆-haloalkyl and oxy-C₁-C₃-alkyleneoxy;
R³ is halogen, cyano, C₁-C₄-alkyl, C₂-C₄-alkenyl, C₂-C₄-alkynyl, C₃-C₆-cycloalkyl, C₁-C₄-alkoxy, C₃-C₄-alkenyloxy, C₃-C₄-alkynyloxy, C₁-C₆-alkylthio, di-(C₁-C₆-alkyl)amino or C₁-C₆-alkylamino, where the alkyl, alkenyl and alkynyl radicals of R³ may be substituted by halogen, cyano, nitro, C₁-C₂-alkoxy or C₁-C₄-alkoxycarbonyl;
X is a group -CH-R^{a}-, -N-R^{b}-, -O- or -S-;
R^{a} may be hydrogen, halogen, C₁-C₆-alkyl, C₁-C₆-alkoxy, cyano or C₁-C₆-alkoxycarbonyl;
R^{b} is hydrogen, C₁-C₆-alkyl or C₃-C₆-cycloalkyl;
T is a group -CH-R^{a}- ;
p is an integer from 1 to 4;
Y is a group -CH-R^{a}- or -N-R^{b}-'
o is 0 or 1;
Z is O, S or a group N(R^{c})
R^{c} is hydrogen, C₁-C₆-alkyl or C₁-C₆-alkoxy.

2. The 2-substituted pyrimidine of the formula I according to claim 1 where
n is an integer from 1 to 3, where at least one substituent L is located in the ortho-position on the phenyl ring;
L is halogen, cyano, methyl, methoxy, -C(=O)-O-A, -C(=O)-N(A')A, C(A')(=N-OA), N(A')A, N(A')-C(=O)-A,
A,A' independently of one another are hydrogen, C₁-C₆-alkyl, C₂-C₆-alkenyl, C₂-C₆-alkynyl, phenyl, where the organic radicals may be partially or fully halogenated or may be substituted by C₁-C₄-alkoxy; or A and A' together with the atoms to which they are attached are a five-or six-membered saturated heterocycle which comprises one or two heteroatoms from the group consisting of O, N and S;
where the aliphatic groups of the radical definitions of L for their part may be partially or fully halogenated;
R¹,R² independently of one another are C₁-C₆-alkyl, C₂-C₆-alkenyl, C₂-C₆-alkynyl, C₁-C₆-haloalkyl, C₂-C₆-haloalkenyl or C₂-C₆-haloalkynyl;
R² may additionally be hydrogen;
¹ and R² Rmay also, together with the nitrogen atom to which they are attached, form a saturated or unsaturated five- or six-membered ring which may be interrupted by an ether -(-O-) or by a further amino -(-N(R^{a})- group, where R^{a} is hydrogen or C₁-C₆-alkyl, and/or may comprise one or more substituents from the group consisting of halogen, C₁-C₆-alkyl, C₁-C₆-haloalkyl and oxy-C₁-C₃-alkyleneoxy;
R³ is halogen, cyano, C₁-C₄-alkyl, C₁-C₄-alkoxy or C₁-C₄-haloalkyl;
X is a group -CH-R^{a}- ;
R^{a} is hydrogen or C₁-C₃-alkyl,
p is an integer from 2 to 4 and
o s 0.

3. The 2-substituted pyrimidine according to claim 1 where R³ is chlorine, cyano, methyl or methoxy.

4. The 2-substituted pyrimidine according to claim 1 where
X is a group -CH-R^{a}-;
R^{a} is hydrogen, halogen, cyano or C₁-C₃-alkyl;
T is a group -CH-R^{a}- ;
p is an integer from 1 to 3;
Y is a group -N-R^{b}
R^{b} is hydrogen or C₁-C₃-alkyl and
o is 1.

5. The 2-substituted pyrimidine according to any of claims 1 to 4 in which the phenyl group substituted by Lₙ is the group B 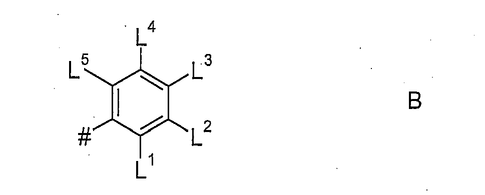 where # is the point of attachment to the pyrimidine skeleton and
L¹ is fluorine, chlorine, CH₃ or CF₃;
L², L⁴ independently of one another are hydrogen, CH₃ or fluorine;
L³ is hydrogen, fluorine, chlorine, cyano, nitro, CH₃, SCH₃, OCH₃, SO₂CH₃, NH-C(=O)CH₃, N(CH₃)-C(=O)CH₃ or COOCH₃ and
L⁵ is hydrogen, fluorine, chlorine or CH₃.

6. A process for preparing the compounds IA according to claim 1 by reacting the sulfone II with the nucleophile III, where the index p is 2 to 5, under basic conditions to give the lactam IA.

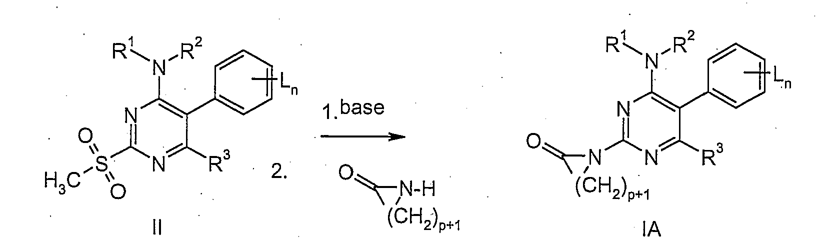

7. A process for preparing the compounds IB according to claim 1 by reacting the hydrazine IV with a chloroalkylcarbonyl chloride, where the index p is 2 or 3, to give the compound V and subsequent cyclization to give the pyrazolone IB.

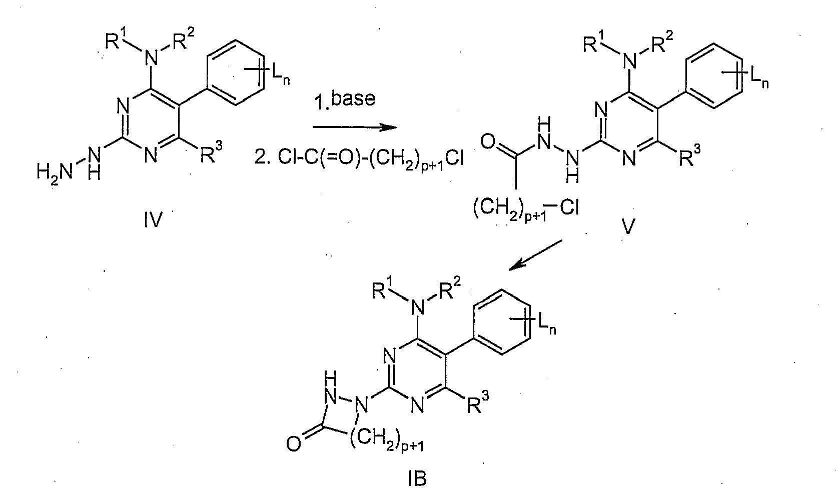

8. A pesticidal composition which comprises a solid or liquid carrier and a compound of the formula I according to claim 1.

9. A method for controlling phytopathogenic harmful fungi which comprises treating the fungi or the materials, plants, the soil or the seeds to be protected against fungal attack with an effective amount of a compound of the formula I according to claim 1.

## Revendications

1. Pyrimidines 2-substituées de la formule I : 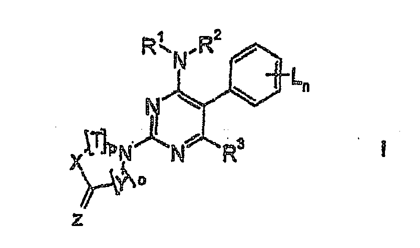 dans laquelle les substituants ont la signification suivante :
n un nombre entier de 1 à 5,
L un halogène, des groupes cyano, cyanato (OCN), alkyle en C₁ à C₈, alcényle en C₂ à C₈, alcynyle en C₂ à C₈, alcoxy en C₁ à C₆, alcényloxy en C₂ à C₈, alcynyloxy en C₂ à C₈, cycloalkyle en C₃ à C₆, cycloalcényle en C₄ à C₆, cycloalkyloxy en C₃ à C₆, cycloalcényloxy en C₄ à C₆, nitro, -C(=O)-A, -C(=O)-O-A, -C(=O)-N(A')A, C(A')(=N-OA), N(A')A, N(A')-C(=O)-A, N(A")- C(=O)-N(A')A, S(=O)ₘ-A, S(=O)ₘ-O-A ou S(=O)ₘ-N(A')A,
m 0, 1 ou 2,
A,A',A" représentent indépendamment les uns des autres de l'hydrogène ou un radical alkyle en C₁ à C₆, alcényle en C₂ à C₆, alcynyle en C₂ à C₆, cycloalkyle en C₃ à C₈, cycloalcényle en C₃ à C₈, phényle, où les radicaux organiques peuvent être partiellement ou totalement halogénés ou peuvent être substitués par des groupes nitro, cyanato, cyano ou alcoxy en C₁ à C₄, ou bien A et A' représentent ensemble avec les atomes auxquels ils sont liés un hétérocycle saturé à cinq à six membres, partiellement insaturé ou aromatique, contenant de un à quatre hétéroatomes du groupe formé par O, N ou S,
où les groupes aliphatiques des définitions des radicaux de L peuvent de leur côté être partiellement ou totalement halogénés ou porter de un à quatre groupes R^{u} :
R^{U} un groupe cyano, alcoxy en C₁ à C₆, cycloalkyle en C₃ à C₆, alcényloxy en C₂ à C₈, alcynyloxy en C₂ à C₈, cycloalcényle en C₄ à C₆, cycloalkyloxy en C₃ à C₆, cycloalcényloxy en C₄ à C₆, -C(=O)-A, -C(=O)-O-A, -C(=O)-N(A')A, C(A')(=N-OA), N(A')A, N(A')-C(=O)- A, N(A")-C(=O)-N(A')A,
S(=O)ₘ-A,S(=O)ₘ-O-A ou S(=O)ₘ-N(A')A,
R¹, R² représentent indépendamment l'un de l'autre un groupe alkyle en C₁ à C₆, alcényle en C₂ à C₆, alcynyle en C₂ à C₆, cycloalkyle en C₃ à C₆, halogénocycloalkyle en C₃ à C₆, où les groupes aliphatiques des définitions des radicaux de R¹ et R² peuvent de leur côté être partiellement ou totalement halogénés ou porter de un à quatre groupes R^{v},
R^{v} représente un groupe cyano, cycloalkyle en C₃ à C₆, cycloalcényle en C₄ à C₆, hydroxy, alcoxy en C₁ à C₆, alcényloxy en C₂ à C₈, alcynyloxy en C₂ à C₈, cycloalkyloxy en C₃ à C₆, cycloalcényloxy en C₄ à C₆, alkylthio en C₁ à C₆, -C(=O)-A, -C(=O)-O-A, -C(=O)-N (A') A, C(A')(=N-OA), N (A') A, N(A')-C(=O)-A, N(A")-C(=O)-N(A')A, S(=O)ₘ-A, S(=O)ₘ-O-A ou S(=O)ₘ-N(A')A ou phényle, la partie phényle pouvant porter de un à trois radicaux choisis dans le groupe formé par un halogène et des groupes, alkyle en C₁ à C₆, alcényle en C₂ à C₆, alcynyle en C₂ à C₆, cycloalkyle en C₃ à C₆, halogénoalkyle en C₁ à C₆, alcoxy en C₁ à C₆, cyano, nitro, -C(=O)-A, -C(=O)-O-A, -C(=O)-N(A')A, C(A')(=N-OA), N(A')A,
R² peut en outre être de l'hydrogène,
R¹ et R² peuvent aussi former ensemble avec l'atome d'azote auquel ils sont liés un noyau à cinq ou six membres, saturé ou insaturé, qui peut être interrompu par un groupe éther (-0-), carbonyle (C=O)-, thio (-S-), sulfoxyle (-S[=O]-) ou sulfényle (-SO₂-) ou un autre amino (-N(R^{a}), où R^{a} représente de l'hydrogène ou un alkyle en C₁ à C₆, et/ou peut contenir un ou plusieurs substituants choisis dans le groupe formé par un halogène et des groupes alkyle en C₁ à C₆, halogénoalkyle en C₁ à C₆ et oxy-(C₁-C₃)alkylèneoxy,
R³ représente un halogène ou un groupe cyano, alkyle en C₁ à C₄, alcényle en C₂ à C₄, alcynyle en C₂ à C₄, cycloalkyle en C₃ à C₆, alcoxy en C₁ à C₄, alcényloxy en C₃ à C₄, alcynyloxy en C₃ à C₄, alkylthio en C₁ à C₆, di(C₁-C₆)alkylamino ou (C₁-C₆)alkylamino, où les radicaux alkyle, alcényle et alcynyle de R³ peuvent être substitués par de l'halogène ou des groupes cyano, nitro, alcoxy en C₁ à C₂ ou alcoxycarbonyle en C₁ à C₄,
X représente un groupe -CH-R^{a}-, -N-R^{b}-, -O- ou -S-,
R^{a} peut être de l'hydrogène, un halogène ou un groupe alkyle en C₁ à C₆, alcoxy en C₁ à C₆, cyano ou alcoxycarbonyle en C₁ à C₆,
R^{b} est de l'hydrogène ou un groupe alkyle en C₁ à C₆ ou cycloalkyle en C₃ à C₆,
T est un groupe -CH-R^{a}-,
p est un nombre entier de 1 à 4,
Y est un groupe -CH-R^{a}- ou -N-R^{b}-,
o vaut 0 ou 1,
Z représente O, S ou un groupe N(R^{c})
R^{c} est de l'hydrogène ou un groupe alkyle en C₁ à C₆ ou alcoxy en C₁ à C₆,

2. Pyrimidines 2-substituées de la formule I suivant la revendication 1, où
n est un nombre entier de 1 à 3, où au moins un substituant L est en position ortho sur le noyau phényle,
L est un halogène ou un groupe cyano, méthyle, méthoxy, -C(=O)-O-A, -C(=O)-N(A')A, C(A')(=N-OA), N(A')A, N(A')-C(=O)-A,
A,A' représentent indépendamment l'un de l'autre de l'hydrogène ou un radical alkyle en C₁ à C₆, alcényle en C₂ à C₆, alcynyle en C₂ à C₆, phényle, où les radicaux organiques peuvent être partiellement ou totalement halogénés ou être substitués par de l'alcoxy en C₁ à C₄, ou bien A et A' représentent, ensemble avec les atomes auxquels ils sont liés, un hétérocycle saturé à cinq à six membres, contenant un ou deux hétéroatomes du groupe formé par 0, N ou S,
où les groupes aliphatiques des définitions des radicaux de L peuvent de leur côté être partiellement ou totalement halogénés,
R¹, R² représentent indépendamment l'un de l'autre un groupe alkyle en C₁ à C₆, alcényle en C₂ à C₆, alcynyle en C₂ à C₆, halogénoalkyle en C₁ à C₆, halogénoalcényle en C₂ à C₆ ou halogénoalcynyle en C₂ à C₆,
R² peut en outre être de l'hydrogène,
R¹ et R peuvent aussi former, ensemble avec l'atome d'azote auquel ils sont liés, former un noyau saturé ou insaturé à cinq ou six membres, qui peut être interrompu par un groupe éther (-O-) ou un autre groupe amino -(-N(R^{a}), où R^{a} représente de l'hydrogène ou un alkyle en C₁ à C₆, et/ou peut contenir un ou plusieurs substituants du groupe formé par un halogène et des groupes alkyle en C₁ à C₆, halogénoalkyle en C₁ à C₆, et oxy(C₁-C₃)alkylèneoxy,
R³ représente un halogène, cyano, ou un groupe alkyle en C₁ à C₄, alcoxy en C₁ à C₄ ou halogénoalkyle en C₁ à C₄,
X est un groupe -CH-R^{a}-,
R^{a} est de l'hydrogène ou un alkyle en C₁ à C₃,
p est un nombre entier de 2 à 4 et
o vaut 0.

3. Pyrimidines 2-substituées suivant la revendication 1, où R³ représente du chlore, un groupe cyano, méthyle ou méthoxy.

4. Pyrimidines 2-substituées suivant la revendication 1, où
X est un groupe -CH-R^{a}-,
R^{a} est de l'hydrogène, un halogène ou un groupe cyano ou alkyle en C₁ à C₃,
T est un groupe -CH-R^{a}-,
p est un nombre entier de 1 à 3,
Y est un groupe -N-R^{b},
R^{b} est de l'hydrogène ou un groupe alkyle en C₁ à C₃ et
o vaut 1.

5. Pyrimidines 2-substituées suivant l'une quelconque des revendications 1 à 4, où les groupes phényle substitués par Lₙ représentent le groupe B : 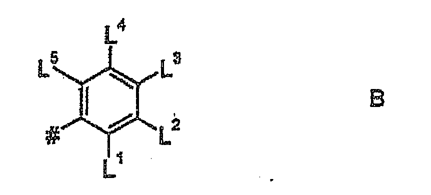 où # est le site de liaison avec le squelette de pyrimidine et
L¹ est du fluor, du chlore ou un groupe CF₃,
L², L⁴ représentent indépendamment l'un de l'autre de l'hydrogène, CH₃ ou du fluor,
L³ est de l'hydrogène, du fluor, du chlore ou un groupe cyano, nitro, CH₃, SCH₃, OCH₃, SO₂CH₃, NH-C(=O)CH₃, N(CH₃)-C(=O)CH₃ ou COOCH₃ et
L⁵ est de l'hydrogène, du fluor, du chlore ou CH₃.

6. Procédé de préparation des composés IA suivant la revendication 1, par réaction de la sulfone II avec le nucléophile III, où l'indice p vaut de 2 à 5, dans des conditions basiques, pour former le lactame IA :

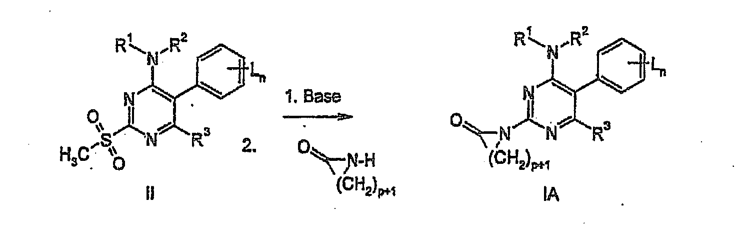

7. Procédé de préparation des composés IB suivant la revendication 1, par réaction de l'hydrazine IV avec un chlorure d'acide chloroalkylcarboxylique,
où l'indice p vaut 2 ou 3, pour former le composé V et cyclisation subséquente pour former la pyrazolone IB :

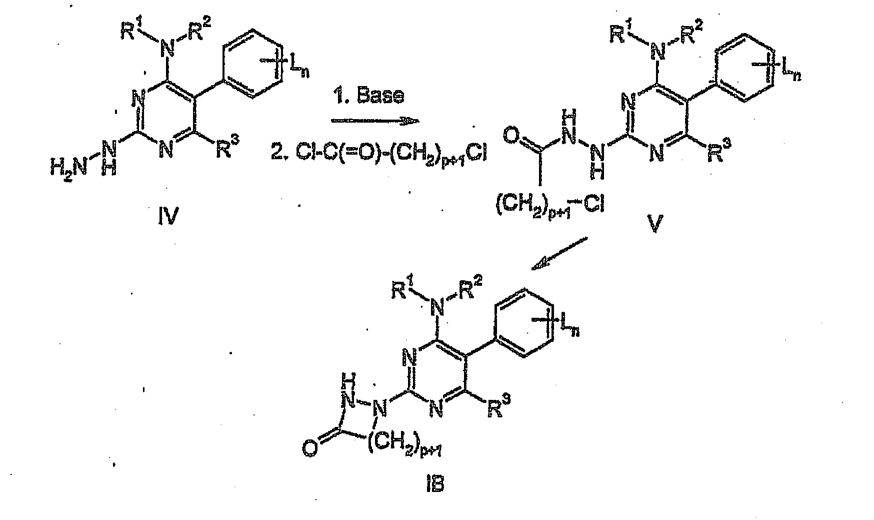

8. Agent pesticide contenant un excipient solide ou liquide et un composé de la formule I suivant la revendication 1.

9. Procédé de lutte contre des champignons nuisibles phytopathogènes, **caractérisé en ce que** les champignons ou les matériaux, plantes, sols ou semences à protéger d'une attaque fongique sont traités par une quantité efficace d'un composé de la formule I suivant la revendication 1.
